# EUROPEAN PATENT APPLICATION

(11) **EP 1 516 930 A1**
(43) Date of publication of application: **23.03.2005**
(21) Application number: 03020506.6
(22) Date of filing: 16.09.2003
(51) Int. Cl.: C12N 15/12, C07K 14/47, G01N 33/68, C07K 16/18, A61K 39/00, A01K 67/027

(54) **Cellular model of tauopathies for lead identification and drug discovery**

(71) Applicant: Georg-August Universität Göttingen, 37075 Göttingen (DE)
(72) Inventor: Wouters, Fred S., Dr., 37075 Göttingen (DE); Iliev, Asparouh I., Dr. M.D., 37083 Göttingen (DE)
(74) Representative: Wichmann, Hendrik, Dr.

(57) **Abstract**

The present invention provides polypeptides with modified microtubule binding domains of a tau protein, which polypeptides lead to an increase of neurofibrillary tangles when introduced into a cell. The invention further provides nucleic acid molecules encoding these polypeptides, cells comprising the polypeptides and trangenic animals with cells expressing these polypeptides. The polypeptides of the invention form the basis for animal and cellular models of tau pathology, which form the basis for molecular screens for biomolecules involved in tauopathies, but also for medicaments useful for the treatment of tauopathies.

SEQUENCE LISTING

<110> Universität Göttingen
Georg-August-Universität Göttingen

<120> Cellular model of tauopathies for lead identification and drug discovery

<130> K62304EP

<160> 43

<170> PatentIn version 3.1

<210> 1
<211> 24
<212> PRT
<213> Homo sapiens

<400> 1
Pro Asp Leu Lys Asn Val Lys Ser Lys Ile Gly Ser Thr Glu Asn Leu
1               5                   10                  15

Lys His Gln Pro Gly Gly Gly Lys
            20

<210> 2
<211> 31
<212> PRT
<213> Homo sapiens

<400> 2
Val Gln Ile Ile Asn Lys Lys Leu Asp Leu Ser Asn Val Gln Ser Lys
1               5                   10                  15

Cys Gly Ser Lys Asp Asn Ile Lys His Val Pro Gly Gly Gly Ser
            20                  25                  30

<210> 3

<211>  31

<212>  PRT

<213>  Homo sapiens

<400>  3

Val Gln Ile Val Tyr Lys Pro Val Asp Leu Ser Lys Val Thr Ser Lys
1               5                   10                  15

Cys Gly Ser Leu Gly Asn Ile His His Lys Pro Gly Gly Gly Gln
            20                  25                  30

<210>  4

<211>  32

<212>  PRT

<213>  Homo sapiens

<400>  4

Val Glu Val Lys Ser Glu Lys Leu Asp Phe Lys Asp Arg Val Gln Ser
1               5                   10                  15

Lys Ile Gly Ser Leu Asp Asn Ile Thr His Val Pro Gly Gly Gly Asn
            20                  25                  30

<210>  5

<211>  31

<212>  PRT

<213>  artificial sequence

<220>

<223>  consensus sequence of microtubule binding domains

<400>  5

Val Gln Ile Val Asn Lys Lys Leu Asp Leu Lys Asn Val Gln Ser Lys
1               5                   10                  15

Cys Gly Ser Thr Asp Asn Ile Lys His Gln Pro Gly Gly Gly Asn
            20                  25                  30

<210>  6

<211>  32

<212>  PRT

<213>  bos taurus

<400>  6

Leu Gln Ala Ala Pro Gly Pro Met Pro Asp Leu Lys Asn Val Lys Ser
1               5                   10                  15

Lys Ile Gly Ser Thr Glu Asn Leu Lys His Gln Pro Gly Gly Gly Lys
            20                  25                  30

<210>  7

<211>  32

<212>  PRT

<213>  bos taurus

<400>  7

Val Glu Val Lys Ser Glu Lys Leu Asp Phe Lys Asp Arg Val Gln Ser
1               5                   10                  15

Lys Ile Gly Ser Leu Asp Asn Ile Thr His Val Pro Gly Gly Gly Asn
            20                  25                  30

<210>  8

<211>  31

<212>  PRT

<213>  bos taurus

<400>  8

Val Gln Ile Ile Asn Lys Lys Leu Asp Leu Ser Asn Val Gln Ser Lys
1               5                   10                  15

Cys Gly Ser Lys Asp Asn Ile Lys His Val Pro Gly Gly Gly Ser
            20                  25                  30

<210>  9

<211>  31

<212>  PRT

<213>  bos taurus

<400>  9

Val Gln Ile Val Tyr Lys Pro Val Asp Leu Ser Lys Val Thr Ser Lys
1               5                   10                  15

Cys Gly Ser Leu Gly Asn Ile His His Lys Pro Gly Gly Gly Gln
            20                  25                  30

<210> 10

<211> 31

<212> PRT

<213> homo sapiens

<400> 10

Arg Leu Ile Asn Gln Pro Leu Pro Asp Leu Lys Asn Val Lys Ser Lys
1               5                   10                  15

Ile Gly Ser Thr Asp Asn Ile Lys Tyr Gln Pro Lys Gly Gly Gln
            20                  25                  30

<210> 11

<211> 31

<212> PRT

<213> homo sapiens

<400> 11

Val Gln Ile Val Thr Lys Lys Ile Asp Leu Ser His Val Thr Ser Lys
1               5                   10                  15

Cys Gly Ser Leu Lys Asn Ile Arg His Arg Pro Gly Gly Gly Arg
            20                  25                  30

<210> 12

<211> 32

<212> PRT

<213> homo sapiens

<400> 12

Val Lys Ile Glu Ser Val Lys Leu Asp Phe Lys Glu Lys Val Gln Ala
1               5                   10                  15

Lys Val Gly Ser Leu Asp Asn Ala His His Val Pro Gly Gly Gly Asn
            20                  25                  30

<210> 13

<211>  31

<212>  PRT

<213>  bos taurus


<400>  13

Val Ala Ala Asn Ala Ser Ala Pro Asp Leu Lys Asn Val Arg Ser Lys
1               5                   10                  15

Val Gly Ser Thr Glu Asn Ile Lys His Gln Pro Gly Gly Gly Arg
            20                  25                  30

<210>  14

<211>  31

<212>  PRT

<213>  bos taurus


<400>  14

Val Gln Ile Gln Asn Lys Lys Val Asp Ile Ser Lys Val Ser Ser Lys
1               5                   10                  15

Cys Gly Ser Lys Ala Asn Ile Lys His Lys Pro Gly Gly Gly Asp
            20                  25                  30

<210>  15

<211>  31

<212>  PRT

<213>  homo sapiens


<400>  15

Val Gln Ile Val Ser Lys Lys Val Ser Tyr Ser His Ile Gln Ser Lys
1               5                   10                  15

Cys Gly Ser Lys Asp Asn Ile Lys His Val Pro Gly Gly Gly Asn
            20                  25                  30

<210>  16

<211>  31

<212>  PRT

<213>  rattus norvegicus

<400> 16

Val Arg Ile Leu Asn Lys Lys Met Asp Phe Ser Lys Val Gln Ser Arg
1               5                   10                  15

Cys Gly Ser Lys Asp Asn Ile Lys His Ser Ala Gly Gly Gly Asn
            20                  25                  30

<210> 17

<211> 30

<212> PRT

<213> artificial sequence

<220>

<223> mutant TBR M1 derived from human tau, amino acids 274-304

<400> 17

Lys Val Gln Ile Asn Ile Lys Leu Asp Leu Ser Asn Val Gln Ser Lys
1               5                   10                  15

Cys Gly Ser Lys Asp Asn Ile Lys His Val Pro Gly Gly Gly
            20                  25                  30

<210> 18

<211> 30

<212> PRT

<213> artificial sequence

<220>

<223> mutant TBR M2, derived from human tau, amino acids 305-335

<400> 18

Ser Val Gln Ile Tyr Val Lys Val Asp Leu Ser Lys Val Thr Ser Lys
1               5                   10                  15

Cys Gly Ser Leu Gly Asn Ile His His Lys Pro Gly Gly Gly
            20                  25                  30

<210> 19

<211> 31

<212> PRT

<213> artificial sequence

<220>

<223>  mutant TBR M3 derived from human tau, amino acids 336-367

<400>  19

Gln Val Glu Val Lys Ile Lys Leu Asp Phe Lys Asp Arg Val Gln Ser
1               5                   10                  15

Lys Ile Gly Ser Leu Asp Asn Ile Thr His Val Pro Gly Gly Gly
            20                  25                  30

<210>  20

<211>  250

<212>  PRT

<213>  homo sapiens


<400>  20

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205

Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met
                245                 250

<210> 21

<211> 221

<212> PRT

<213> homo sapiens

<400> 21

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Ala Glu Glu Ala Gly Ile Gly
65                  70                  75                  80

Asp Thr Pro Ser Leu Glu Asp Glu Ala Ala Gly His Val Thr Gln Ala
                85                  90                  95

Arg Met Val Ser Lys Ser Lys Asp Gly Thr Gly Ser Asp Asp Lys Lys
            100                 105                 110

Ala Lys Gly Ala Asp Gly Lys Thr Lys Ile Ala Thr Pro Arg Gly Ala
        115                 120                 125

Ala Pro Pro Gly Gln Lys Gly Gln Ala Asn Ala Thr Arg Ile Pro Ala
    130                 135                 140

Lys Thr Pro Pro Ala Pro Lys Thr Pro Pro Ser Ser Gly Glu Pro Pro
145                 150                 155                 160

Lys Ser Gly Asp Arg Ser Gly Tyr Ser Ser Pro Gly Ser Pro Gly Thr
                165                 170                 175

Pro Gly Ser Arg Ser Arg Thr Pro Ser Leu Pro Thr Pro Pro Thr Arg
            180                 185                 190

Glu Pro Lys Lys Val Ala Val Val Arg Thr Pro Pro Lys Ser Pro Ser
        195                 200                 205

Ser Ala Lys Ser Arg Leu Gln Thr Ala Pro Val Pro Met
    210                 215                 220

<210>  22

<211>  192

<212>  PRT

<213>  homo sapiens


<400>  22

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Ala Glu Glu Ala
        35                  40                  45

Gly Ile Gly Asp Thr Pro Ser Leu Glu Asp Glu Ala Ala Gly His Val
    50                  55                  60

Thr Gln Ala Arg Met Val Ser Lys Ser Lys Asp Gly Thr Gly Ser Asp
65                  70                  75                  80

Asp Lys Lys Ala Lys Gly Ala Asp Gly Lys Thr Lys Ile Ala Thr Pro
                85                  90                  95

Arg Gly Ala Ala Pro Pro Gly Gln Lys Gly Gln Ala Asn Ala Thr Arg
            100                 105                 110

Ile Pro Ala Lys Thr Pro Pro Ala Pro Lys Thr Pro Pro Ser Ser Gly
        115                 120                 125

Glu Pro Pro Lys Ser Gly Asp Arg Ser Gly Tyr Ser Ser Pro Gly Ser
    130                 135                 140

Pro Gly Thr Pro Gly Ser Arg Ser Arg Thr Pro Ser Leu Pro Thr Pro
145                 150                 155                 160

Pro Thr Arg Glu Pro Lys Lys Val Ala Val Val Arg Thr Pro Pro Lys
                165                 170                 175

Ser Pro Ser Ser Ala Lys Ser Arg Leu Gln Thr Ala Pro Val Pro Met
            180                 185                 190

<210>  23

<211>  567

<212>  PRT

<213>  homo sapiens

<400>  23

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Glu Pro Glu Ser
        115                 120                 125

Gly Lys Val Val Gln Glu Gly Phe Leu Arg Glu Pro Gly Pro Pro Gly
    130                 135                 140

Leu Ser His Gln Leu Met Ser Gly Met Pro Gly Ala Pro Leu Leu Pro
145                 150                 155                 160

Glu Gly Pro Arg Glu Ala Thr Arg Gln Pro Ser Gly Thr Gly Pro Glu
                165                 170                 175

Asp Thr Glu Gly Gly Arg His Ala Pro Glu Leu Leu Lys His Gln Leu
            180                 185                 190

Leu Gly Asp Leu His Gln Glu Gly Pro Pro Leu Lys Gly Ala Gly Gly
        195                 200                 205

Lys Glu Arg Pro Gly Ser Lys Glu Glu Val Asp Glu Asp Arg Asp Val
    210                 215                 220

Asp Glu Ser Ser Pro Gln Asp Ser Pro Pro Ser Lys Ala Ser Pro Ala
225                 230                 235                 240

Gln Asp Gly Arg Pro Pro Gln Thr Ala Ala Arg Glu Ala Thr Ser Ile
                245                 250                 255

Pro Gly Phe Pro Ala Glu Gly Ala Ile Pro Leu Pro Val Asp Phe Leu
            260                 265                 270

Ser Lys Val Ser Thr Glu Ile Pro Ala Ser Glu Pro Asp Gly Pro Ser
        275                 280                 285

Val Gly Arg Ala Lys Gly Gln Asp Ala Pro Leu Glu Phe Thr Phe His
    290                 295                 300

Val Glu Ile Thr Pro Asn Val Gln Lys Glu Gln Ala His Ser Glu Glu
305                 310                 315                 320

His Leu Gly Arg Ala Ala Phe Pro Gly Ala Pro Gly Glu Gly Pro Glu
                325                 330                 335

Ala Arg Gly Pro Ser Leu Gly Glu Asp Thr Lys Glu Ala Asp Leu Pro
            340                 345                 350

Glu Pro Ser Glu Lys Gln Pro Ala Ala Ala Pro Arg Gly Lys Pro Val
        355                 360                 365

Ser Arg Val Pro Gln Leu Lys Ala Arg Met Val Ser Lys Ser Lys Asp
    370                 375                 380

Gly Thr Gly Ser Asp Asp Lys Lys Ala Lys Thr Ser Thr Arg Ser Ser
385                 390                 395                 400

Ala Lys Thr Leu Lys Asn Arg Pro Cys Leu Ser Pro Lys Leu Pro Thr
                405                 410                 415

Pro Gly Ser Ser Asp Pro Leu Ile Gln Pro Ser Ser Pro Ala Val Cys
            420                 425                 430

Pro Glu Pro Pro Ser Ser Pro Lys His Val Ser Ser Val Thr Ser Arg
        435                 440                 445

Thr Gly Ser Ser Gly Ala Lys Glu Met Lys Leu Lys Gly Ala Asp Gly
    450                 455                 460

Lys Thr Lys Ile Ala Thr Pro Arg Gly Ala Ala Pro Pro Gly Gln Lys
465                 470                 475                 480

Gly Gln Ala Asn Ala Thr Arg Ile Pro Ala Lys Thr Pro Pro Ala Pro
                485                 490                 495

Lys Thr Pro Pro Ser Ser Gly Glu Pro Pro Lys Ser Gly Asp Arg Ser
            500                 505                 510

Gly Tyr Ser Ser Pro Gly Ser Pro Gly Thr Pro Gly Ser Arg Ser Arg
        515                 520                 525

Thr Pro Ser Leu Pro Thr Pro Pro Thr Arg Glu Pro Lys Lys Val Ala
    530                 535                 540

Val Val Arg Thr Pro Pro Lys Ser Pro Ser Ser Ala Lys Ser Arg Leu
545                 550                 555                 560

Gln Thr Ala Pro Val Pro Met
                565

<210>  24

<211>  73

<212>  PRT

<213>  homo sapiens


<400>  24

Lys Lys Ile Glu Thr His Lys Leu Thr Phe Arg Glu Asn Ala Lys Ala
1               5                   10                  15

Lys Thr Asp His Gly Ala Glu Ile Val Tyr Lys Ser Pro Val Val Ser
            20                  25                  30

Gly Asp Thr Ser Pro Arg His Leu Ser Asn Val Ser Ser Thr Gly Ser
        35                  40                  45

Ile Asp Met Val Asp Ser Pro Gln Leu Ala Thr Leu Ala Asp Glu Val
    50                  55                  60

Ser Ala Ser Leu Ala Lys Gln Gly Leu
65                  70

<210>  25

<211>  441

<212>  PRT

<213>  Homo sapiens


<400>  25

<210>  26

<211>  372

<212>  PRT

<213>  Mus musculus


<400>  26

Met Ala Asp Pro Arg Gln Glu Phe Asp Thr Met Glu Asp His Ala Gly
1               5                   10                  15

Asp Tyr Thr Leu Leu Gln Asp Gln Glu Gly Asp Met Asp His Gly Leu
            20                  25                  30

Lys Ala Glu Glu Ala Gly Ile Gly Asp Thr Pro Asn Gln Glu Asp Gln
        35                  40                  45

Ala Ala Gly His Val Thr Gln Ala Arg Val Ala Ser Lys Asp Arg Thr
    50                  55                  60

Gly Asn Asp Glu Lys Lys Ala Lys Gly Ala Asp Gly Lys Thr Gly Ala
65                  70                  75                  80

Lys Ile Ala Thr Pro Arg Gly Ala Ala Ser Pro Ala Gln Lys Gly Thr
                85                  90                  95

Ser Asn Ala Thr Arg Ile Pro Ala Lys Thr Thr Pro Ser Pro Lys Thr
            100                 105                 110

Pro Pro Gly Ser Gly Glu Pro Pro Lys Ser Gly Glu Arg Ser Gly Tyr
        115                 120                 125

Ser Ser Pro Gly Ser Pro Gly Thr Pro Gly Ser Arg Ser Arg Thr Pro
    130                 135                 140

Ser Leu Pro Thr Pro Pro Thr Arg Glu Pro Lys Lys Val Ala Val Val
145                 150                 155                 160

Arg Thr Pro Pro Lys Ser Pro Ser Ala Ser Lys Ser Arg Leu Gln Thr
                165                 170                 175

Ala Pro Val Pro Met Pro Asp Leu Lys Asn Val Gly Ser Lys Ile Gly
            180                 185                 190

Ser Thr Glu Asn Leu Lys His Gln Pro Gly Gly Gly Lys Val Gln Ile
        195                 200                 205

Ile Asn Lys Lys Leu Asp Leu Ser Asn Val Gln Ser Lys Cys Gly Ser
    210                 215                 220

Lys Asp Asn Ile Lys His Val Pro Gly Gly Gly Ser Val Gln Ile Val
225                 230                 235                 240

Tyr Lys Pro Val Asp Leu Ser Lys Val Thr Ser Lys Cys Gly Ser Leu
                245                 250                 255

Gly Asn Ile His His Lys Pro Gly Gly Gly Gln Val Glu Val Lys Ser
            260                 265                 270

Glu Lys Leu Asp Phe Lys Asp Arg Val Gln Ser Lys Ile Gly Ser Leu
        275                 280                 285

Asp Asn Ile Thr His Val Pro Gly Gly Gly Asn Lys Lys Ile Glu Thr
    290                 295                 300

His Lys Leu Thr Phe Arg Glu Asn Ala Lys Ala Lys Thr Asp His Gly
305                 310                 315                 320

Ala Glu Ile Val Tyr Lys Ser Pro Val Val Ser Gly Asp Thr Ser Pro
                325                 330                 335

Arg His Leu Ser Asn Val Ser Ser Thr Gly Ser Ile Asp Met Val Asp
            340                 345                 350

Ser Pro Gln Leu Ala Thr Leu Ala Asp Glu Val Ser Ala Ser Leu Ala
        355                 360                 365

Lys Gln Gly Leu
    370

<210>  27

<211>  430

<212>  PRT

<213>  Mus musculus

<400>  27

Met Ala Asp Pro Arg Gln Glu Phe Asp Thr Met Glu Asp His Ala Gly
1               5                   10                  15

Asp Tyr Thr Leu Leu Gln Asp Gln Glu Gly Asp Met Asp His Gly Leu
            20                  25                  30

Lys Glu Ser Pro Pro Gln Pro Pro Ala Asp Asp Gly Ala Glu Glu Pro
        35                  40                  45

Gly Ser Glu Thr Ser Asp Ala Lys Ser Thr Pro Thr Ala Glu Asp Val
    50                  55                  60

Thr Ala Pro Leu Val Asp Glu Arg Ala Pro Asp Lys Gln Ala Ala Ala
65                  70                  75                  80

Gln Pro His Thr Glu Ile Pro Glu Gly Ile Thr Ala Glu Glu Ala Gly
                85                  90                  95

Ile Gly Asp Thr Pro Asn Gln Glu Asp Gln Ala Ala Gly His Val Thr
            100                 105                 110

Gln Ala Arg Val Ala Ser Lys Asp Arg Thr Gly Asn Asp Glu Lys Lys
        115                 120                 125

Ala Lys Gly Ala Asp Gly Lys Thr Gly Ala Lys Ile Ala Thr Pro Arg
    130                 135                 140

Gly Ala Ala Ser Pro Ala Gln Lys Gly Thr Ser Asn Ala Thr Arg Ile
145                 150                 155                 160

Pro Ala Lys Thr Thr Pro Ser Pro Lys Thr Pro Pro Gly Ser Gly Glu
                165                 170                 175

Pro Pro Lys Ser Gly Glu Arg Ser Gly Tyr Ser Ser Pro Gly Ser Pro
            180                 185                 190

Gly Thr Pro Gly Ser Arg Ser Arg Thr Pro Ser Leu Pro Thr Pro Pro
        195                 200                 205

Thr Arg Glu Pro Lys Lys Val Ala Val Val Arg Thr Pro Pro Lys Ser
    210                 215                 220

Pro Ser Ala Ser Lys Ser Arg Leu Gln Thr Ala Pro Val Pro Met Pro
225                 230                 235                 240

Asp Leu Lys Asn Val Arg Ser Lys Ile Gly Ser Thr Glu Asn Leu Lys
                245                 250                 255

His Gln Pro Gly Gly Gly Lys Val Gln Ile Ile Asn Lys Lys Leu Asp
            260                 265                 270

Leu Ser Asn Val Gln Ser Lys Cys Gly Ser Lys Asp Asn Ile Lys His
        275                 280                 285

Val Pro Gly Gly Gly Ser Val Gln Ile Val Tyr Lys Pro Val Asp Leu
    290                 295                 300

Ser Lys Val Thr Ser Lys Cys Gly Ser Leu Gly Asn Ile His His Lys
305                 310                 315                 320

Pro Gly Gly Gly Gln Val Glu Val Lys Ser Glu Lys Leu Asp Phe Lys
                325                 330                 335

Asp Arg Val Gln Ser Lys Ile Gly Ser Leu Asp Asn Ile Thr His Val
            340                 345                 350

Pro Gly Gly Gly Asn Lys Lys Ile Glu Thr His Lys Leu Thr Phe Arg
        355                 360                 365

Glu Asn Ala Lys Ala Lys Thr Asp His Gly Ala Glu Ile Val Tyr Lys
    370                 375                 380

Ser Pro Val Val Ser Gly Asp Thr Ser Pro Arg His Leu Ser Asn Val
385                 390                 395                 400

Ser Ser Thr Gly Ser Ile Asp Met Val Asp Ser Pro Gln Leu Ala Thr
                405                 410                 415

Leu Ala Asp Glu Val Ser Ala Ser Leu Ala Lys Gln Gly Leu
            420                 425                 430

<210>  28

<211>  374

<212>  PRT

<213>  Rattus norvegicus

<400>  28

Met Ala Glu Pro Arg Gln Glu Phe Asp Thr Met Glu Asp Gln Ala Gly
1               5                   10                  15

Asp Tyr Thr Met Leu Gln Asp Gln Glu Gly Asp Met Asp His Gly Leu
            20                  25                  30

Lys Ala Glu Glu Ala Gly Ile Gly Asp Thr Pro Asn Met Glu Asp Gln
        35                  40                  45

Ala Ala Gly His Val Thr Gln Ala Arg Val Ala Gly Val Ser Lys Asp
    50                  55                  60

Arg Thr Gly Asn Asp Glu Lys Lys Ala Lys Gly Ala Asp Gly Lys Thr
65                  70                  75                  80

Gly Ala Lys Ile Ala Thr Pro Arg Gly Ala Ala Thr Pro Gly Gln Lys
                85                  90                  95

Gly Thr Ser Asn Ala Thr Arg Ile Pro Ala Lys Thr Thr Pro Ser Pro
            100                 105                 110

Lys Thr Pro Pro Gly Ser Gly Glu Pro Pro Lys Ser Gly Glu Arg Ser
        115                 120                 125

Gly Tyr Ser Ser Pro Gly Ser Pro Gly Thr Pro Gly Ser Arg Ser Arg
    130                 135                 140

Thr Pro Ser Leu Pro Thr Pro Pro Thr Arg Glu Pro Lys Lys Val Ala
145                 150                 155                 160

Val Val Arg Thr Pro Pro Lys Ser Pro Ser Ala Ser Lys Ser Arg Leu
                165                 170                 175

Gln Thr Ala Pro Val Pro Met Pro Asp Leu Lys Asn Val Arg Ser Lys
            180                 185                 190

Ile Gly Ser Thr Glu Asn Leu Lys His Gln Pro Gly Gly Gly Lys Val
        195                 200                 205

Gln Ile Ile Asn Lys Lys Leu Asp Leu Ser Asn Val Gln Ser Lys Cys
    210                 215                 220

Gly Ser Lys Asp Asn Ile Lys His Val Pro Gly Gly Gly Ser Val Gln
225                 230                 235                 240

Ile Val Tyr Lys Pro Val Asp Leu Ser Lys Val Thr Ser Lys Cys Gly
                245                 250                 255

Ser Leu Gly Asn Ile His His Lys Pro Gly Gly Gly Gln Val Glu Val
            260                 265                 270

Lys Ser Glu Lys Leu Asp Phe Lys Asp Arg Val Gln Ser Lys Ile Gly
        275                 280                 285

Ser Leu Asp Asn Ile Thr His Val Pro Gly Gly Gly Asn Lys Lys Ile
    290                 295                 300

Glu Thr His Lys Leu Thr Phe Arg Glu Asn Ala Lys Ala Lys Thr Asp
305                 310                 315                 320

His Gly Ala Glu Ile Val His Lys Ser Pro Val Val Ser Gly Asp Thr
                325                 330                 335

Ser Pro Arg His Leu Ser Asn Val Ser Ser Thr Gly Ser Ile Asp Met
            340                 345                 350

Val Asp Ser Pro Gln Leu Ala Thr Leu Ala Asp Glu Val Ser Ala Ser
        355                 360                 365

Leu Ala Lys Gln Gly Leu
    370

<210>  29

<211>  432

<212>  PRT

<213>  Rattus norvegicus


<400>  29

Met Ala Glu Pro Arg Gln Glu Phe Asp Thr Met Glu Asp Gln Ala Gly
1               5                   10                  15

Asp Tyr Thr Met Leu Gln Asp Gln Glu Gly Asp Met Asp His Gly Leu
            20                  25                  30

Lys Glu Ser Pro Pro Gln Pro Pro Ala Asp Asp Gly Ser Glu Glu Pro
        35                  40                  45

Gly Ser Glu Thr Ser Asp Ala Lys Ser Thr Pro Thr Ala Glu Asp Val
    50                  55                  60

Thr Ala Pro Leu Val Glu Glu Arg Ala Pro Asp Lys Gln Ala Thr Ala
65                  70                  75                  80

Gln Ser His Thr Glu Ile Pro Glu Gly Thr Thr Ala Glu Glu Ala Gly
                85                  90                  95

Ile Gly Asp Thr Pro Asn Met Glu Asp Gln Ala Ala Gly His Val Thr
            100                 105                 110

Gln Ala Arg Val Ala Gly Val Ser Lys Asp Arg Thr Gly Asn Asp Glu
        115                 120                 125

Lys Lys Ala Lys Gly Ala Asp Gly Lys Thr Gly Ala Lys Ile Ala Thr
    130                 135                 140

Pro Arg Gly Ala Ala Thr Pro Gly Gln Lys Gly Thr Ser Asn Ala Thr
145                 150                 155                 160

Arg Ile Pro Ala Lys Thr Thr Pro Ser Pro Lys Thr Pro Pro Gly Ser
                165                 170                 175

Gly Glu Pro Pro Lys Ser Gly Glu Arg Ser Gly Tyr Ser Ser Pro Gly
            180                 185                 190

Ser Pro Gly Thr Pro Gly Ser Arg Ser Arg Thr Pro Ser Leu Pro Thr
        195                 200                 205

Pro Pro Thr Arg Glu Pro Lys Lys Val Ala Val Val Arg Thr Pro Pro
    210                 215                 220

Lys Ser Pro Ser Ala Ser Lys Ser Arg Leu Gln Thr Ala Pro Val Pro
225                 230                 235                 240

Met Pro Asp Leu Lys Asn Val Arg Ser Lys Ile Gly Ser Thr Glu Asn
                245                 250                 255

Leu Lys His Gln Pro Gly Gly Gly Lys Val Gln Ile Ile Asn Lys Lys
            260                 265                 270

Leu Asp Leu Ser Asn Val Gln Ser Lys Cys Gly Ser Lys Asp Asn Ile
        275                 280                 285

Lys His Val Pro Gly Gly Gly Ser Val Gln Ile Val Tyr Lys Pro Val
    290                 295                 300

Asp Leu Ser Lys Val Thr Ser Lys Cys Gly Ser Leu Gly Asn Ile His
305                 310                 315                 320

His Lys Pro Gly Gly Gly Gln Val Glu Val Lys Ser Glu Lys Leu Asp
                325                 330                 335

Phe Lys Asp Arg Val Gln Ser Lys Ile Gly Ser Leu Asp Asn Ile Thr
            340                 345                 350

His Val Pro Gly Gly Gly Asn Lys Lys Ile Glu Thr His Lys Leu Thr
        355                 360                 365

Phe Arg Glu Asn Ala Lys Ala Lys Thr Asp His Gly Ala Glu Ile Val
    370                 375                 380

Tyr Lys Ser Pro Val Val Ser Gly Asp Thr Ser Pro Arg His Leu Ser
385                 390                 395                 400

Asn Val Ser Ser Thr Gly Ser Ile Asp Met Val Asp Ser Pro Gln Leu
                405                 410                 415

Ala Thr Leu Ala Asp Glu Val Ser Ala Ser Leu Ala Lys Gln Gly Leu
            420                 425                 430

<210>  30

<211>  448

<212>  PRT

<213>  Bos taurus


<400>  30

Met Ala Glu Pro Arg Gln Glu Phe Asp Val Met Glu Asp His Ala Gln
1               5                   10                  15

Gly Asp Tyr Thr Leu Gln Asp Gln Glu Gly Asp Met Asp Pro Gly Leu
            20                  25                  30

Lys Glu Ser Pro Leu Gln Thr Pro Ala Asp Asp Gly Ser Glu Glu Pro
        35                  40                  45

Gly Ser Glu Thr Ser Asp Ala Lys Ser Thr Pro Thr Ala Glu Asp Ala
    50                  55                  60

Thr Ala Pro Leu Val Asp Glu Gly Ala Pro Gly Glu Gln Ala Ala Ala
65                  70                  75                  80

Gln Ala Pro Ala Glu Ile Pro Glu Gly Thr Ala Ala Glu Glu Ala Gly
                85                  90                  95

Ile Gly Asp Thr Ser Asn Leu Glu Asp Gln Ala Ala Gly His Val Thr
            100                 105                 110

Gln Ala Arg Met Val Ser Lys Gly Lys Asp Gly Thr Gly Pro Asp Asp
        115                 120                 125

Lys Lys Thr Lys Gly Ala Asp Gly Lys Pro Gly Thr Lys Ile Ala Thr
    130                 135                 140

Pro Arg Gly Ala Ala Pro Pro Gly Gln Lys Gly Gln Ala Asn Ala Thr
145                 150                 155                 160

Arg Ile Pro Ala Lys Thr Thr Pro Thr Pro Lys Thr Ser Pro Ala Thr
                165                 170                 175

Met Gln Val Gln Lys Lys Pro Pro Pro Ala Gly Ala Lys Ser Glu Arg
            180                 185                 190

Gly Glu Ser Gly Lys Ser Gly Asp Arg Ser Gly Tyr Ser Ser Pro Gly
        195                 200                 205

Ser Pro Gly Thr Pro Gly Ser Arg Ser Arg Thr Pro Ser Leu Pro Thr
    210                 215                 220

Pro Pro Thr Arg Glu Pro Lys Lys Val Ala Val Val Arg Thr Pro Pro
225                 230                 235                 240

Lys Ser Pro Ser Ala Ala Lys Ser Arg Leu Gln Ala Ala Pro Gly Pro
                245                 250                 255

Met Pro Asp Leu Lys Asn Val Lys Ser Lys Ile Gly Ser Thr Glu Asn
            260                 265                 270

Leu Lys His Gln Pro Gly Gly Gly Lys Val Gln Ile Ile Asn Lys Lys
        275                 280                 285

Leu Asp Leu Ser Asn Val Gln Ser Lys Cys Gly Ser Lys Asp Asn Ile
    290                 295                 300

Lys His Val Pro Gly Gly Gly Ser Val Gln Ile Val Tyr Lys Pro Val
305                 310                 315                 320

Asp Leu Ser Lys Val Thr Ser Lys Cys Gly Ser Leu Gly Asn Ile His
                325                 330                 335

His Lys Pro Gly Gly Gly Gln Val Glu Val Lys Ser Glu Lys Leu Asp
            340                 345                 350

Phe Lys Asp Arg Val Gln Ser Lys Ile Gly Ser Leu Asp Asn Ile Thr
        355                 360                 365

His Val Pro Gly Gly Gly Asn Lys Lys Ile Glu Thr His Lys Leu Thr
    370                 375                 380

Phe Arg Glu Asn Ala Lys Ala Lys Thr Asp His Gly Ala Glu Ile Val
385                 390                 395                 400

Tyr Lys Ser Pro Val Val Ser Gly Asp Thr Ser Pro Arg His Leu Ser
                405                 410                 415

Asn Val Ser Ser Thr Gly Ser Ile Asp Met Val Asp Ser Pro Gln Leu
            420                 425                 430

Ala Thr Leu Ala Asp Glu Val Ser Ala Ser Leu Ala Lys Gln Gly Leu
        435                 440                 445

<210>  31

<211>  361

<212>  PRT

<213>  Drosophila melanogaster


<400>  31

Met Ala Asp Val Leu Glu Lys Ser Ser Leu Leu Asp Ala Val Pro Pro
1               5                   10                  15

Leu Gly Asp Pro His Pro Pro Leu Pro His Gln Gln Leu Gln Gln Glu
            20                  25                  30

Ala Ala Ala Ala Ala Ala Ala Asn Ala Ala Pro Pro Ala Pro Pro Gln
        35                  40                  45

Gln Gln Gln Pro Pro Pro His Gln Leu Gln Gln Gln Gln Pro Gln Gln
    50                  55                  60

Gln Gln Leu Gln Gln Lys Pro Ala Asn Ala Arg Ala Asn Gln Asp Gln
65                  70                  75                  80

Lys Glu Gly Asp Asn Asp Ser Gly Val Asp Glu Ser Thr Gln Glu Lys
                85                  90                  95

Asp Arg Asn Gly Pro Asn Ser Pro Ser Ser Pro Val Lys Thr Pro Thr
            100                 105                 110

Ser Thr Ser Ser Lys Pro Asp Lys Ser Gly Thr Ser Arg Pro Pro Ser
        115                 120                 125

Ala Thr Pro Ser Asn Lys Ser Ala Pro Lys Ser Arg Ser Ala Ser Lys
    130                 135                 140

Asn Arg Leu Leu Leu Lys Thr Pro Glu Pro Glu Pro Val Lys Lys Val
145                 150                 155                 160

Pro Met Asn Lys Val Gln Val Gly His Ala Pro Ser Pro Asn Leu Lys
                165                 170                 175

Ala Val Arg Ser Lys Ile Gly Ser Leu Asp Asn Ala Thr Tyr Lys Pro
            180                 185                 190

Gly Gly Gly His Val Lys Ile Glu Ser Lys Lys Ile Asp Ile Lys Ala
        195                 200                 205

Ala Pro Arg Ile Glu Ala Lys Asn Asp Lys Tyr Met Pro Lys Gly Gly
    210                 215                 220

Glu Lys Lys Ile Val Thr Thr Lys Leu Gln Trp Asn Ala Lys Ser Lys
225                 230                 235                 240

Ile Gly Ser Leu Glu Asn Ala Ala His Lys Pro Gly Gly Gly Asp Lys
                245                 250                 255

Lys Ile Glu Thr Leu Lys Met Asp Phe Lys Asp Lys Ala Lys Pro Lys
            260                 265                 270

Val Gly Ser Thr Ala Asn Val Lys His Gln Pro Gly Gly Gly Asp Ile
        275                 280                 285

Lys Ile Gln Thr Gln Lys Leu Glu Ile Lys Ala Gln Ser Lys Val Gly
    290                 295                 300

Ser Leu Asp Asn Val Lys His Lys Pro Gly Gly Gly Glu Lys Lys Ile
305                 310                 315                 320

Phe Asp Asp Lys Asp Tyr Leu Lys Asn Val Glu His Ser Val Ala Leu
                325                 330                 335

Thr Thr Pro Pro Thr Gln Ser Pro Leu Pro Ser Met Thr Ala Ser Gly
            340                 345                 350

Ala Asp Glu Asn Leu Asn Gln Gln Ser
        355                 360

<210>  32

<211>  453

<212>  PRT

<213>  Caenorhabditis elegans

<400> 32

Met Ser Thr Pro Gln Ser Glu Pro Gly Ser Glu Pro Gly Ser Pro Gly
1               5                   10                  15

Glu Asn Glu Asn Ser Met Glu Thr Gln Tyr Ser Gln Gly Ser Phe Pro
            20                  25                  30

Glu Val Ala Val Glu Pro Glu Pro Glu Pro Glu Pro Glu Pro Glu Pro
        35                  40                  45

Glu Pro Glu Pro Glu Pro Glu Pro Glu Pro Glu Pro Glu Pro Lys Pro
    50                  55                  60

Lys Pro Glu Val Glu Pro Val Pro Gln Pro Glu Pro Glu Pro Glu Tyr
65                  70                  75                  80

Gln Pro Glu Pro Glu Pro Glu Pro Glu Val Glu Pro Glu Pro Glu Pro
                85                  90                  95

Glu Pro Val Pro Glu Pro Glu Pro Glu Pro Glu Pro Glu Pro Glu Pro
            100                 105                 110

Val Val Glu Lys Glu Glu Glu Val Val Val Glu Ser Pro Pro Arg Glu
        115                 120                 125

Gln Glu Pro Glu Lys Ser Gly Lys Ser Lys Pro Ser Ser Pro Ile Pro
    130                 135                 140

Asp Ala Pro Thr Met Glu Asp Ile Ala Pro Arg Glu Leu Glu Ser Leu
145                 150                 155                 160

Asn Phe Ser Glu Thr Ser Gly Thr Ser Asp Gln Gln Ala Asp Arg Ile
                165                 170                 175

Met Gln Asn Asn Glu Asn Glu Arg Val Glu Glu Lys Lys Gln Met Ser
            180                 185                 190

Pro Thr Pro Ser Gln Pro Gln His Lys Thr Pro Gln Arg Ser Gly Ile
        195                 200                 205

Arg Pro Pro Thr Ala Ile Leu Arg Gln Pro Lys Pro Ile Pro Ala Ser
    210                 215                 220

Leu Pro Arg Pro Ala Thr Ala Thr Pro Ser Ser Gln Arg Ala Ile Ser
225                 230                 235                 240

Thr Pro Arg Gln Thr Ala Ser Thr Ala Pro Ser Pro Arg Pro Ile Ser
                245                 250                 255

Lys Met Ser Arg Glu Arg Ser Asp Val Gln Lys Ser Thr Ser Thr Arg
            260                 265                 270

Ser Ile Asp Asn Val Gly Arg Met Thr Pro Lys Val Asn Ala Lys Phe
        275                 280                 285

Val Asn Val Lys Ser Lys Val Gly Ser Val Thr Asn His Lys Ala Gly
    290                 295                 300

Gly Gly Asn Val Glu Ile Phe Ser Glu Lys Arg Leu Tyr Asn Ala Gln
305                 310                 315                 320

Ser Lys Val Gly Ser Leu Lys Asn Ala Thr His Val Ala Gly Gly Gly
                325                 330                 335

Asn Val Gln Ile Glu Asn Arg Lys Leu Asp Phe Ser Ala Ala Ser Pro
            340                 345                 350

Lys Val Gly Ser Lys Thr Asn Tyr Gln Pro Ala Lys Ser Asp Val Lys
        355                 360                 365

Ile Val Ser Glu Lys Leu Thr Trp Gln Ala Lys Ser Lys Val Gly Ser
    370                 375                 380

Met Asp Asn Ala Ala His Lys Pro Ala Gly Gly Asn Val Gln Ile Leu
385                 390                 395                 400

Ser Gln Lys Leu Asn Trp Lys Ala Glu Ser Lys Val Gly Ser Lys Asp
                405                 410                 415

Asn Met Asn His Arg Pro Gly Gly Gly Asn Val Gln Ile Phe Asp Glu
            420                 425                 430

Lys Ile Arg Tyr Val Ser Thr Asp Ser Ser Arg Asn His Ser Thr Leu
        435                 440                 445

Asp Ile Ser Ser Leu
    450

<210>  33

<211>  413

<212>  PRT

<213>  Caenorhabditis elegans


<400>  33

Met Ser Thr Pro Gln Ser Glu Pro Gly Ser Glu Pro Gly Ser Pro Gly
1               5                   10                  15

Glu Asn Glu Asn Ser Met Glu Thr Gln Tyr Ser Gln Gly Ser Phe Pro
            20                  25                  30

Glu Val Ala Val Glu Pro Glu Pro Glu Pro Glu Pro Glu Pro Glu Pro
        35                  40                  45

Glu Pro Glu Pro Glu Pro Glu Pro Glu Pro Glu Pro Glu Pro Lys Pro
    50                  55                  60

Lys Pro Glu Val Glu Pro Val Pro Gln Pro Glu Pro Glu Pro Glu Tyr
65                  70                  75                  80

Gln Pro Glu Pro Glu Pro Glu Pro Glu Val Glu Pro Glu Pro Glu Pro
                85                  90                  95

Glu Pro Val Pro Glu Pro Glu Pro Glu Pro Glu Pro Glu Pro Glu Pro
            100                 105                 110

Val Val Glu Lys Glu Glu Glu Val Val Val Glu Ser Pro Pro Arg Glu
        115                 120                 125

Gln Glu Pro Glu Lys Ser Gly Lys Ser Lys Pro Ser Ser Pro Ile Pro
    130                 135                 140

Asp Ala Pro Thr Met Glu Asp Ile Ala Pro Arg Glu Leu Glu Ser Leu
145                 150                 155                 160

Asn Phe Ser Glu Thr Ser Gly Thr Ser Asp Gln Gln Ala Asp Arg Ile
                165                 170                 175

Met Gln Asn Asn Glu Asn Glu Arg Val Glu Glu Lys Lys Gln Met Ser
            180                 185                 190

Pro Thr Pro Ser Gln Pro Gln His Lys Thr Pro Gln Arg Ser Gly Ile
        195                 200                 205

Arg Pro Pro Thr Ala Ile Leu Arg Gln Pro Lys Pro Ile Pro Ala Ser
    210                 215                 220

Leu Pro Arg Pro Ala Thr Ala Thr Pro Ser Ser Gln Arg Ala Ile Ser
225                 230                 235                 240

Thr Pro Arg Gln Thr Ala Ser Thr Ala Pro Ser Pro Arg Pro Ile Ser
                245                 250                 255

Lys Met Ser Arg Glu Arg Ser Asp Val Gln Lys Ser Thr Ser Thr Arg
            260                 265                 270

Ser Ile Asp Asn Val Gly Arg Met Thr Pro Lys Val Asn Ala Lys Phe
        275                 280                 285

Val Asn Val Lys Ser Lys Val Gly Ser Val Thr Asn His Lys Ala Gly
    290                 295                 300

Gly Gly Asn Val Glu Ile Phe Ser Glu Lys Arg Leu Tyr Asn Ala Gln
305                 310                 315                 320

Ser Lys Val Gly Ser Leu Lys Asn Ala Thr His Val Ala Gly Gly Gly
                325                 330                 335

Asn Val Gln Ile Glu Asn Arg Lys Leu Asp Phe Ser Ala Ala Ser Pro
            340                 345                 350

Lys Val Gly Ser Lys Thr Asn Tyr Gln Pro Ala Lys Ser Asp Val Lys
        355                 360                 365

Ile Val Ser Glu Lys Leu Thr Trp Gln Ala Lys Ser Lys Val Gly Ser
    370                 375                 380

Met Asp Asn Ala Ala His Lys Pro Ala Gly Gly Asn Val Gln Arg Ser
385                 390                 395                 400

Gly Ser Lys Ser Thr Lys Asn Asp Asp Gly His Asn Asn
                405                 410

<210>  34

<211>  440

<212>  PRT

<213>  Artificial Sequence


<220>

<223>  mutant tau M2

<400>  34

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205

Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met Pro Asp Leu Lys Asn Val
                245                 250                 255

Lys Ser Lys Ile Gly Ser Thr Glu Asn Leu Lys His Gln Pro Gly Gly
            260                 265                 270

Gly Lys Val Gln Ile Ile Asn Lys Lys Leu Asp Leu Ser Asn Val Gln
        275                 280                 285

Ser Lys Cys Gly Ser Lys Asp Asn Ile Lys His Val Pro Gly Gly Gly
    290                 295                 300

Ser Val Gln Ile Tyr Val Lys Val Asp Leu Ser Lys Val Thr Ser Lys
305                 310                 315                 320

Cys Gly Ser Leu Gly Asn Ile His His Lys Pro Gly Gly Gly Gln Val
                325                 330                 335

Glu Val Lys Ser Glu Lys Leu Asp Phe Lys Asp Arg Val Gln Ser Lys
            340                 345                 350

Ile Gly Ser Leu Asp Asn Ile Thr His Val Pro Gly Gly Gly Asn Lys
        355                 360                 365

Lys Ile Glu Thr His Lys Leu Thr Phe Arg Glu Asn Ala Lys Ala Lys
    370                 375                 380

Thr Asp His Gly Ala Glu Ile Val Tyr Lys Ser Pro Val Val Ser Gly
385                 390                 395                 400

Asp Thr Ser Pro Arg His Leu Ser Asn Val Ser Ser Thr Gly Ser Ile
                405                 410                 415

Asp Met Val Asp Ser Pro Gln Leu Ala Thr Leu Ala Asp Glu Val Ser
            420                 425                 430

Ala Ser Leu Ala Lys Gln Gly Leu
        435                 440

<210>  35

<211>  439

<212>  PRT

<213>  Artificial Sequence

<220>

<223>  mutant tau M23

<400>  35

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205

Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met Pro Asp Leu Lys Asn Val
                245                 250                 255

Lys Ser Lys Ile Gly Ser Thr Glu Asn Leu Lys His Gln Pro Gly Gly
            260                 265                 270

Gly Lys Val Gln Ile Ile Asn Lys Lys Leu Asp Leu Ser Asn Val Gln
        275                 280                 285

Ser Lys Cys Gly Ser Lys Asp Asn Ile Lys His Val Pro Gly Gly Gly
    290                 295                 300

Ser Val Gln Ile Tyr Val Lys Val Asp Leu Ser Lys Val Thr Ser Lys
305                 310                 315                 320

Cys Gly Ser Leu Gly Asn Ile His His Lys Pro Gly Gly Gly Gln Val
                325                 330                 335

Glu Val Lys Ile Lys Leu Asp Phe Lys Asp Arg Val Gln Ser Lys Ile
            340                 345                 350

Gly Ser Leu Asp Asn Ile Thr His Val Pro Gly Gly Gly Asn Lys Lys
        355                 360                 365

Ile Glu Thr His Lys Leu Thr Phe Arg Glu Asn Ala Lys Ala Lys Thr
    370                 375                 380

Asp His Gly Ala Glu Ile Val Tyr Lys Ser Pro Val Val Ser Gly Asp
385                 390                 395                 400

Thr Ser Pro Arg His Leu Ser Asn Val Ser Ser Thr Gly Ser Ile Asp
                405                 410                 415

Met Val Asp Ser Pro Gln Leu Ala Thr Leu Ala Asp Glu Val Ser Ala
            420                 425                 430

Ser Leu Ala Lys Gln Gly Leu
        435

<210>  36

<211>  439

<212>  PRT

<213>  Artificial Sequence

<220>

<223>  mutant tau m12

<400>  36

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205

Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met Pro Asp Leu Lys Asn Val
                245                 250                 255

Lys Ser Lys Ile Gly Ser Thr Glu Asn Leu Lys His Gln Pro Gly Gly
            260                 265                 270

Gly Lys Val Gln Ile Asn Ile Lys Leu Asp Leu Ser Asn Val Gln Ser
        275                 280                 285

Lys Cys Gly Ser Lys Asp Asn Ile Lys His Val Pro Gly Gly Gly Ser
    290                 295                 300

Val Gln Ile Tyr Val Lys Val Asp Leu Ser Lys Val Thr Ser Lys Cys
305                 310                 315                 320

Gly Ser Leu Gly Asn Ile His His Lys Pro Gly Gly Gly Gln Val Glu
                325                 330                 335

Val Lys Ser Glu Lys Leu Asp Phe Lys Asp Arg Val Gln Ser Lys Ile
            340                 345                 350

Gly Ser Leu Asp Asn Ile Thr His Val Pro Gly Gly Gly Asn Lys Lys
        355                 360                 365

Ile Glu Thr His Lys Leu Thr Phe Arg Glu Asn Ala Lys Ala Lys Thr
    370                 375                 380

Asp His Gly Ala Glu Ile Val Tyr Lys Ser Pro Val Val Ser Gly Asp
385                 390                 395                 400

Thr Ser Pro Arg His Leu Ser Asn Val Ser Ser Thr Gly Ser Ile Asp
                405                 410                 415

Met Val Asp Ser Pro Gln Leu Ala Thr Leu Ala Asp Glu Val Ser Ala
            420                 425                 430

Ser Leu Ala Lys Gln Gly Leu
        435

<210>  37

<211>  438

<212>  PRT

<213>  Artificial Sequence

<220>

<223>  mutant tau M123

<400>  37

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Ala Arg Met Val
        115                 120                 125

Ser Lys Ser Lys Asp Gly Thr Gly Ser Asp Asp Lys Lys Ala Lys Gly
    130                 135                 140

Ala Asp Gly Lys Thr Lys Ile Ala Thr Pro Arg Gly Ala Ala Pro Pro
145                 150                 155                 160

Gly Gln Lys Gly Gln Ala Asn Ala Thr Arg Ile Pro Ala Lys Thr Pro
                165                 170                 175

Pro Ala Pro Lys Thr Pro Pro Ser Ser Gly Glu Pro Pro Lys Ser Gly
            180                 185                 190

Asp Arg Ser Gly Tyr Ser Ser Pro Gly Ser Pro Gly Thr Pro Gly Ser
        195                 200                 205

Arg Ser Arg Thr Pro Ser Leu Pro Thr Pro Pro Thr Arg Glu Pro Lys
    210                 215                 220

Lys Val Ala Val Val Arg Thr Pro Pro Lys Ser Pro Ser Ser Ala Lys
225                 230                 235                 240

Ser Arg Leu Gln Thr Ala Pro Val Pro Met Pro Asp Leu Lys Asn Val
                245                 250                 255

Lys Ser Lys Ile Gly Ser Thr Glu Asn Leu Lys His Gln Pro Gly Gly
            260                 265                 270

Gly Lys Val Gln Ile Asn Ile Lys Leu Asp Leu Ser Asn Val Gln Ser
        275                 280                 285

Lys Cys Gly Ser Lys Asp Asn Ile Lys His Val Pro Gly Gly Gly Ser
    290                 295                 300

Val Gln Ile Tyr Val Lys Val Asp Leu Ser Lys Val Thr Ser Lys Cys
305                 310                 315                 320

Gly Ser Leu Gly Asn Ile His His Lys Pro Gly Gly Gly Gln Val Glu
                325                 330                 335

Val Lys Ile Lys Leu Asp Phe Lys Asp Arg Val Gln Ser Lys Ile Gly
            340                 345                 350

Ser Leu Asp Asn Ile Thr His Val Pro Gly Gly Gly Asn Lys Lys Ile
        355                 360                 365

Glu Thr His Lys Leu Thr Phe Arg Glu Asn Ala Lys Ala Lys Thr Asp
    370                 375                 380

His Gly Ala Glu Ile Val Tyr Lys Ser Pro Val Val Ser Gly Asp Thr
385                 390                 395                 400

Ser Pro Arg His Leu Ser Asn Val Ser Ser Thr Gly Ser Ile Asp Met
                405                 410                 415

Val Asp Ser Pro Gln Leu Ala Thr Leu Ala Asp Glu Val Ser Ala Ser
            420                 425                 430

Leu Ala Lys Gln Gly Leu
        435

<210>  38

<211>  54

<212>  DNA

<213>  artificial sequence



<220>

<223>  oligonucleotide used in the construction of mutant M1 - sense str
       and

<400>  38
caccagccgg gaggcgggaa ggtgcagata aatatcaagc tggatcttag caac          54

<210>  39

<211>  54

<212>  DNA

<213>  artificial sequence



<220>

<223>  oligonucleotide used in the construction of mutant M1 - antisense
        strand

<400>  39
gttgctaaga tccagcttga tatttatctg caccttcccg cctcccggct ggtg          54

<210>  40

<211>  36

<212>  DNA

<213>  artificial sequence



<220>

<223>  oligo used in the construction of mutant M1 - sense strand

<400>  40
cacgtcccgg gaggcggcag tgtgcaaata tacgtc                              36

<210>  41

<211>  36

<212>  DNA

<213>  artificial sequence



<220>

<223>  oligo used in the construction of mutant M2 - antisense strand

<400>  41
cttgctcagg tcaactttga cgtatatttg cacact                                36

<210>  42

<211>  57

<212>  DNA

<213>  artificial sequence

<220>

<223>  oligo used in the construction of mutant M3 - sense strand

<400>  42
cataaaccag gaggtggcca ggtggaagta aaaatcaagc ttgacttcaa ggacaga         57

<210>  43

<211>  57

<212>  DNA

<213>  artificial sequence

<220>

<223>  oligo used in the contruction of mutant M3 - antisense strand

<400>  43
tctgtccttg aagtcaagct tgatttttac ttccacctgg ccacctcctg gtttatg         57

## Description

### BACKGROUND OF THE INVENTION

The present invention provides tau mutants which lead to formation of tau aggregates, which exhibit the properties of neurofibrillary tangles when introduced into a cell. Therefore even in the context of non-neuronal cells we will henceforth refer to these aggregates as neurofibrillary tangles (NFTs). These tau mutants form the basis for improved animal and cellular models of tau pathology, which form the basis for molecular screens for biomolecules involved in tauopathies and medicaments useful for the treatment of tauopathies.

Neuronal tau aggregation, first described by Kidd, M. (1963) (Paired Helical Filaments in electron microscopy in Alzheimers Disease. *Nature* **197,** 192-193), is a typical morphological finding in Alzheimer's disease (AD) and other neurodegenerative disorders such as Frontotemporal dementia with Parkinsonism linked to chromosome 17 (FTDP-17), Pick's disease (PiD), progressive supranuclear palsy (PSP), amyotrophic lateral sclerosis/Parkinsonism-dementia complex of Guam (ALS/PDC) and corticobasal degeneration (CBD), summarized under the name "tauopathies". These disorders share somatic and neuritic tau protein aggregates which are commonly named "neurofibrillary tangles" (NFT). NFTs can be visualized in affected tissues by silver impregnation or by staining with Thioflavin S, Thiazin Red or Congo Red. In NFTs the tau protein is hyper-phosphorylated. In this neurotoxic state the NFTs spread predominantly in layers III and V of the neocortex and in the entorhinal cortex of the limbic system, as well as throughout the hippocampus.

A distinct special dissociation between the distribution of NFTs and senile amyloid plaques (SP), also found in AD, exists. Further analysis of the pathomorphological findings has revealed that predominantly the quantity of NFTs, and to a lesser extent that of SPs, correlates with the clinical score of AD (Arriagada *et al., Neurology* **42,** 631-639; Bierer *et* *al., Arch. Neurol*. **52,** 81-88). In March 2003, a first pathomorphological evidence of a fatal patient outcome after amyloid-β-peptide vaccination was published in *Nature Medicine* (Nicoll *et al., Nat. Med*. **9,** 448-452), highlighting several important conclusions in terms of the pathological events that trigger dementia. After the treatment the amyloid burden was dramatically reduced, which did not correspond to substantial improvement of cognitive scoring. Additionally, the amount of NFTs was virtually unaffected. Apparently, any further treatment should address the formation and the effects of both SPs and NFTs, which probably play a synergistic role in disease progression.

The normal physiological role of tau is inferred from its microtubule localization and from numerous biochemical experiments and is thought to be the stabilization of the microtubule system and the regulation of microtubule transport. Also a role in neurite extension has been suggested.

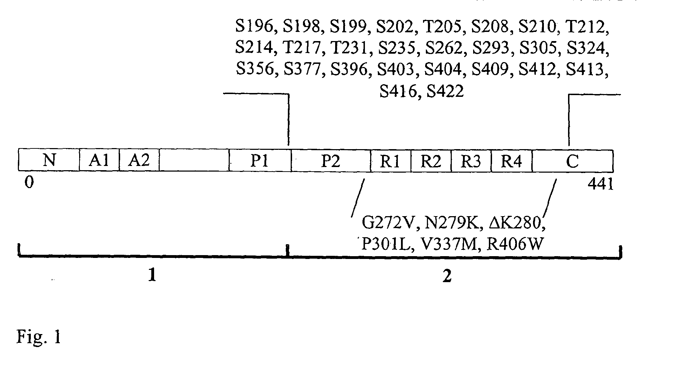

The physiological properties of the tau protein are related to its structure and to the presence of four microtubule-binding repeats. Tau stabilizes microtubules (MT) and promotes neurite elongation in neurons by virtue of these microtubule-binding repeats. Figure 1 also shows phosphorylation epitopes (P1, P2 and C-terminal (upper scale)), microtubule-binding domains (R1-R4), as well as the major pathological mutations within R1-R4, known from inherited tauopathies (lower scale).

In tauopathies like, for example, AD, the protein tau has been shown to undergo a number of transitions that make it "pathological". Most of these biochemical transitions have been reproduced on purified protein in cell-free conditions but, to date, no good cellular model exists that mimics the behavior of tau in tauopathies like, for example, AD. These progressive biochemical transitions comprise: a) probably reduced binding of tau to microtubules, b) hyperphosphorylation at sites S202/T205 (numbers according to tau441 (SEQ ID NO:25), T212/S214, S396/S404, etc., c) conformational change as detected by conformation-sensitive antibodies, d) disulfide-bridge mediated dimerization by cysteine oxidation, e) oligomerization of tau to form fibrillation "kernels" or "seeds", f) polymerization of tau onto these kernels to form "proto-fibrils", g) extended polymerization to form 15-20 nm thick fibrils with overcrossing at every 80 nm that can be visualized by electron microscopy as "paired helical filaments" (PHFs). (Spillantini and Goedert (1998) *Trends Neurosci.* **21**, 428-433.) These PHFs presumably have a β-sheet core. (Berriman *et al*., (2003) *PNAS* 100:9034-9038.) In affected tissues, the NFTs can be detected by their increased β-sheet content by the use of reagents that preferentially bind to β-sheet structures, *i*.*e*. Thioflavin, Congo Red, Thiazin Red.

A number of mutations have been identified in the tau protein that are the cause of familial tauopathies, like, for example, FTDP-17. Most of these mutations (see Figure 1) are located in or around the microtubule-binding repeats and are thus thought to destabilize microtubule binding, giving rise to higher concentrations of soluble tau polypeptides, which might then reassemble with themselves. The other mutations are thought to have an unknown effect on folding that enhances the chance for polymerization or change the ratio of the six forms of tau normally expressed in the brain.

All attempts to produce animal models that faithfully represent tauopathies like, for example, AD or FTDP-17, today are based on overexpression of tau carrying one or more of these mutations. Major mutations are R406W (Tatebayashi *et al*. (2002) *Proc. Natl. Acad. Sci. USA* **99,** 13896-13901), P301L (Lewis *et al*. (2000) *Nat. Genet*. **25,** 402-405), V337M (Tanemura *et al*. (2001) *Neurobiol*. *Dis.* **8,** 1036-1045) and some others. These mutations, however, yield unsatisfactory phenotypes when introduced in a cellular or animal model system. Moreover, the lack of understanding on the mechanism by which these mutations might cause tau protein aggregation makes these mutations poor tools. Furthermore, the fact that naturally occurring familial mutations are evolutionarily maintained means that the mutations themselves do not represent the "optimal" changes to tau for the induction of fibrillation, as this would be lethal at a far earlier age, *i*.*e*. it would affect the individual's chance of reproduction. This, in turn, has consequences for the animal models that are based on these mutations. These animals still have to reach a significant age (normally 1-1.5 years for rodents!) before the effects of the mutations are visible and the effects are often very mild as the neurons have the possibility to compensate for some of the toxic effects if they take place at a sufficiently slow pace.

This is not a situation that lends itself for drug screening, where one would like to have rapidly progressing pathology in relatively young animals in order to rapidly obtain detailed information on the potential efficacy of drug candidates. One of the best models of tauopathies available today is the overexpression of the human tau R406W mutant in the *Drosophila* fruitfly (Wittmann *et al*. (2001) *Science* **293,** 711-714). Another fruitfly model utilizes normal human tau, overexpressed together with *shaggy* (a *Drosophila* analogue of glycogen synthase kinase-3) (Jackson *et al*. (2002) *Neuron* **34,** 509-519). These animals age fast and are readily available in large numbers. However, due to the partial understanding of the mechanism-of-action of natural tau mutations, these animals never show fibril structures, even though neurons die in an age-dependent fashion. Additionally, some of the typical pathomorphological features of the pathological tau are not present.

Moreover, the need of 20-25 days until symptoms occur in flies still limits the suitability of the model for high-throughoutput screening of drugs. Though mechanistically interesting in that it demonstrates that toxicity can precede fibrillation, this is not an optimal model for drug screening as not all aspects of tau pathology have been reproduced.

Also the present cellular models of tau pathology are unsatisfactory. Relatively few attempts for modeling exist, most of them using okadaic acid to phosphorylate tau so as to cause partial tau aggregation. However, okadaic acid by itself causes apoptosis within 24 hours after application (Perez *et al*. (2002) *Eur. J. Biochem.* **269,** 1484-1489). The usage of natural mutants does not reveal any apparent phenotype, while co-expression together with GSK3b and other proteins (Sato *et al*. (2002) *J. Biol. Chem.* **277,** 42060-42065) significantly perturb the cellular biochemistry, again without achieving a satisfactory phenotype. A recent paper by Fath *et al*. (2002, *J. Neurosci*. **22,** 9733-9741) describes cellular toxicity of a pseudophosphorylation tau mutant, but the disadvantages of the model are the lack of normal function and physiological distribution, which was demonstrated, as well as a complete loss of phosphorylation-dependent regulation of tau dynamics, that is vital for its physiology and toxicity. In neuroblastoma cells after cell cycle synchronization with nocodazole some pathological phosphoepitopes (AT8, AT100, etc.) are detectable, although the very synchronization of cell division phases already represents a significant manipulation of normal cellular physiology (Delobel *et al*. (2002) *J*. *Neurochem.* **83,** 412-420). Nevertheless, the paper of Delobel *et al*. demonstrates the importance of cell division-related kinases to the process of pathological tau phosphorylation.

In the absence of suitable animal models or cellular models for tau pathology the present inventors explored ways of improving such animal or cellular models of tauopathies.

### SUMMARY OF THE INVENTION

The present invention provides tau mutants which lead to an increase of NFTs when introduced into a cell. These tau mutants form the basis for improved animal and cellular models of tau pathology, which form the basis for molecular screens for biomolecules involved in tauopathies and medicaments useful for the treatment of tauopathies.

### DEFINITIONS

A "polypeptide" as used herein is a molecule comprising more than 30, and in particular more than 35, 40, 45 or even more than 50 amino acids, but less than 10,000, in particular less than 9,000, 8,000, 7,000, 6,000, 5,000, 4,000, 3,000, or 2,000, most preferably less than 1,500 amino acids. Polypeptides are usually linear amino acid polymers, wherein the individual amino acids are linked to one another via peptide bonds. Also, polypeptides which contain a low percentage, e.g. less than 5%, 3% or even only up to 1% of modified or non-natural amino acids, are encompassed. Polypeptides can be further modified by chemical modification, e.g. by phosphorylation of serine, threonine, or tyrosine residues, or by glycosylation, e.g. of asparagines or serine residues.

"Peptide" as used herein is a molecule comprising less than 30 amino acids, but preferably more than 4, 5, 6, 7, 8, or even more than 9 amino acids.

The term "A microtubule-binding domain" as used herein is a polypeptide sequence which can be identified as a tubulin-binding repeat (TBR) (pfam00418 according to the Conserved Domain Database of the NCBI). The consensus sequence of a TBR is given below: SEQ ID NO. 5: VQIVNKKLDLKNVQSKCGSTDNIKHQPGGGN (31 amino acids; from N-terminus to C-terminus). Identification can be performed, by searching a polypeptide sequence of interest with the NCBI Protein-protein Blast [Blastp] against the Swissprot protein database. The Blastp programm then identifies those regions within a given polypeptide sequence with homology to conserved domains, and it can be checked whether some of them are putative tubulin-binding repeats.

Typically, TBRs have a length of from 20 to 40 amino acids, more typically from 25 to 35 amino acids and most typically from 29 to 33 amino acids. They can contain a region at their C-terminus which has the characteristics of a flexible linker; this can be, as it is the case in naturally occurring TBRs, a stretch of four amino acids wherein at least three of the four amino acids of the sequence PGGG are present. A TBR may be further characterized by having the following sequence characteristics (the numeration is according to the 31 aa consensus sequence SEQ ID NO. 5, given above):
His or Tyr at position 25; Ile, Leu, Val or Ala at position 23; Asn at position 22; Ser at position 19; Gly at position 18; Lys or Arg at position 16; Val, Ile Leu or Ala at position 13. A polypeptide sequence fulfilling this sequence requirement is herein called a polypeptide "having the TBR-framework".

A "modified microtubule-binding domain" is derived from a TBR by mutagenesis and has a polypeptide sequence which is not encoded as such by the genome of a naturally occurring species, in particular a polypeptide sequence that is not identical to one comprised by those polypeptide sequences of the gene bank database as of September 12, 2003 with a naturally occurring species identified as its source. This means that a "modified microtubule-binding domain" is a polypeptide sequence that does not occur as such within a protein in nature, but can be, and in particular was, produced by laboratory manipulations, such as genetic engineering techniques or chemical synthesis. Examples of modified microtubule-binding domains are such polypeptide sequences which, compared to any naturally occurring TBR, have a mutation, in particular a single, a double, a triple or a quadruple deletion (by way of example, which is relevant for the terms "double" and "triple" as well: both a deletion of four sequential amino acids and of four individual amino acids are considered a quadruple deletion), and/or a single, a double, a triple or a quadruple (see explanation given above) insertion, and/or a single, a double, a triple, a quadruple (see explanation given above) or multiple (e.g. of 5, 6, 7 or even 8 amino acid residues) substitutions. Deletions and substitutions, deletions and insertions, insertions and substitutions as well as deletions, substitutions and insertions can be combined in a "modified microtubule binding domain".

Preferably, however, the modified microtubule-binding domain displays at least 30% identity and 60% homology with any naturally occurring tubulin-binding repeat, in particular with any one of the sequences named SEQ ID No's 1, 2, 3, 4, 6, 7, 8, 9, 10, 11, 12, 13, 14, 15 or 16.

Preferably, the "modified microtubule-binding domain" also has the TBR-framework.

A polypeptide has "at least X% identity with" a microtubule-binding domain of a tau protein, and in particular a human tau protein, if, when a 31 amino acid stretch of its polypeptide sequence is aligned with any one of the microtubule-binding domains of a tau protein, and in particular with any of the sequences listed in Table 1 a) or b), the amino acid identity between those two aligned sequences is X%. X can be 30 or more. Preferably X is at least 40, more preferably at least 50, even more preferably at least 60 and most preferably at least 70%.

Preferably, the nature of the amino acid residue change by which the polypeptide with at least X% identity to one of the reference sequences differs from said reference sequence is a semi-conservative or conservative and more preferably a conservative amino acid residue exchange.

| Amino acid | Conservative substitution | Semi-conservative substitution |
|---|---|---|
| A | G;S;T | N;V;C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y; F; K; R | L; M; A |
| I | V;L;M;A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure.

As used herein, "FRET" relates to the phenomenon known as "fluorescence resonance energy transfer". The principle of FRET has been described for example in J.R. Lakowicz, "Principles of Fluorescence Spectroscopy", 2^{nd} Ed. Plenum Press, New York, 1999. Briefly, FRET can occur if the emission spectrum of a first chromophore (donor chromophore or FRET-donor) overlaps with the absorption spectrum of a second chromophore (acceptor chromophore or FRET-acceptor), so that excitation by lower-wavelength light of the donor chromophore is followed by transfer of the excitation energy to the acceptor chromophore. A prerequisite for this phenomenon is the very close proximity of both chromophores. A result of FRET is the decrease/loss of emission by the donor chromophore while at the same time emission by the acceptor chromophore is observed. A further result of FRET is shortening of the duration of the donor excited state as detected by a reduction in the fluorescence lifetime. A pair of 2 chromophores which can interact in the above described manner is called a "donor-acceptor-pair" for FRET.

A "chromophore" as used herein is that part of a molecule responsible for its light-absorbing and light-emitting properties. A chromophore can be an independent chemical entity. Chromophores can be low-molecular substances, for example, the indocyanin chromophores CY3, CY3.5, Cy5, Cy7 (available from Amersham International plc, GB), fluorescein, coumarin and Alexa Fluor fluorophores (for example, from Molecular Probes). But chromophores can also be fluorescent proteins, like P4-3, EGFP, S65T, BFP, CFP, YFP, to name but a few. The latter are also commercially available in a variety of forms, for example in the context of expression constructs.

When reference is made herein to "human tau protein" (hs-tau), particularly references with regard to amino acid residue numbering, this relates to the CNS specific microtubule-associated adult isoform of the protein tau, which is a 441 amino acid polypeptide having the Accession Number AAC04279 (NCBI protein database). For ease of reference, its sequence is further given herein as SEQ ID NO:25. The overall structure of hs-tau is as follows: An N-terminal projection domain is followed by four microtubule-binding repeats: amino acids 251 to 274 form an imperfect, but identifiable, tubulin-binding repeat (TBR) which misses the 10 most N-terminal amino acids of a typical TBR, while amino acids 275-305, 306-336 and 337-368 form the three TBRs of this particular tau isoform.

As used herein, a flexible linker is a sequence of amino acid residues which confers structural flexibility. As described above, this can be a stretch of four amino acids wherein at least three of the four amino acids of the sequence PGGG are present. Another example is a "glycine-rich linker", which comprises a peptide sequence with two or more glycine residues or a peptide sequence with alternating glycine and serine residues, in particular the amino acid sequences Gly-Gly, Gly-Ser-Gly, and Gly-Gly-Ser-Gly-Gly. With regard to glycine-rich linkers reference is made to Witchlow M. *et al*. (1993) *Prot. Engineering,* **6**:989-995.
"Tauopathies" are disorders and diseases, and in particular neurological disorders and diseases, characterized by the presence of neuronal tau aggregation, in particular the presence of neurofilbrillary tangles. Typical tauopathies are Alzheimer's disease (AD) and other neurodegenerative disorders such as Frontotemporal dementia with Parkinsonism linked to chromosome 17 (FTDP-17), Pick's disease (PiD), progressive supranuclear palsy (PSP), amyotrophic lateral sclerosis/ Parkinsonism-dementia complex of Guam (ALS/PDC) and corticobasal degeneration (CBD).
A "small chemical compound" as used herein is a molecule with a molecular weight from 30 D - 5 kD, preferably from 100 D - 2 kD. A "small organic chemical molecule" as used herein further comprises at least one carbon atom, one hydrogen atom and one oxygen atom. Such small chemical compounds can, e.g., be provided by using available combinatorial libraries. These molecules can be screened for using the methods provided herein and can then be identified in accordance with procedures known in the art.

### DETAILED DESCRIPTION OF THE PREFERRED EMBODIMENTS

The present invention relates to mutants of the tau protein which lead to a formation of neurofibrillary tangles when introduced into a cell. This is achieved by such tau mutants which show increased aggregation kinetics and/or an increased overall extent of aggregation due to the presence of modified microtubule-binding domains therein. The modified microtubule-binding domains confer increased aggregation kinetics and/or an increased overall extent of aggregation to a polypeptide comprising them due to regions of alternating polar and non-polar amino acids. The polypeptide of the invention comprises at least one - for example 1, 2, 3, 4, 5, 6, 7 or 8 - modified microtubule-binding domain of a tau protein, preferably a mammalian tau protein, and more preferably a human tau protein, wherein the modified microtubule-binding domain comprises a region of at least 7 alternating polar and non-polar amino acids; that is a sequence: non-polar/polar/non-polar/polar/non-polar/polar/non-polar or polar/non-polar/polar/non-polar/polar/non-polar/polar. Alternatively, the polypeptide of the invention comprises at least two - for example 2, 3, 4, 5, 6, 7 or 8 - microtubule-binding domains of a tau protein, preferably a mammalian tau protein, and more preferably a human tau protein, wherein either one microtubule-binding domain is modified and comprises two region of at least six alternating polar and non-polar amino acids or two microtubule-binding domains are modified and both comprise a region of at least six alternating polar and non-polar amino acids. Alternatively, the polypeptide of the invention comprises at least three - for example 3, 4, 5, 6, 7 or 8 - microtubule-binding domains of a tau protein, preferably a mammalian tau protein and more preferably a human tau protein, wherein either two microtubule-binding domains are modified and one of the modified microtubule-binding domains contains two regions of at least five alternating polar and non-polar amino acids and the second of the microtubule-binding domains comprises one region of at least five alternating polar and non-polar amino acids, or three microtubule-binding domains are modified and all three comprise a region of at least five alternating polar and non-polar amino acids. The common logic linking those polypeptides of the invention with modified microtubule-binding domains is that due to the introduced regions of polar and non-polar amino acids in the microtubule-binding domains, the polypeptides are capable of displaying increased aggregation *in vitro and*/*or in vivo,* for example when present in the cell of a transgenic, non-human animal, and/or *ex vivo,* e.g. when transfected into cells kept in cell culture (as determined in an experiment like in Example 2), and/or show increased neurotoxicity (as determined in an experiment like in Example 5) and/or cell toxicity (as determined in an experiment like in Examples 3 or 4) in comparison to the wild-type human tau protein. It is preferred that a polypeptide of the invention displays al least a level of aggregation that is equal to or higher than the level obtained under identical conditions with mutant M2. It is further preferred that the polypeptide of the invention displays at least a level of aggregation which, when measured in a FRAP-experiment like in Example 3, using the conditions of Example 3, leads to an immobile fraction of at least 20% at a time point 120s after bleaching. It is also preferred that the polypeptide of the invention displays 20% of the neurotoxicity of mutant M123 (as determined 72 hours after transfection in an experiment using the conditions of Example 5 and otherwise identical conditions for the two polypeptides to be compared with one another) and/or 20% of the cell toxicity of mutant M123 (as determined 72 hours after transfection in an experiment using the conditions of Examples 3 or 4 and otherwise identical conditions for the two polypeptides to be compared with one another). Without wishing to be bound by any theory, the introduced regions of alternating polar and non-polar amino acids might work by providing "kernels" or "seeds" for oligomerization of tau to increase fibril formation.

Preferably, the polypeptide of the invention comprises at least one - for example 1, 2, 3, 4, 5, 6, 7 or 8 - modified microtubule-binding domain of a tau protein, wherein the modified microtubule-binding domain comprises a region of 8, 9, 10, 11, 12, 13, 14 or 15 alternating polar and non-polar amino acids, preferably a region of 9, 10, 11, 12 or 13 alternating polar and non-polar amino acids and more preferably a region of 10, 11 or 12 alternating polar and non-polar amino acids. Preferably, the polar residues of said regions of alternating polar and non-polar amino acids are selected from the group consisting of Ser, Arg, Lys, Asp, Glu, Asn, Gln, His, Thr, Tyr, Trp, Gly, Ala and Cys, preferably selected from the group consisting of Ser, Arg, Lys, Asp, Glu, Asn, Gln, and Thr, and more preferably selected from the group consisting of Ser, Lys, Asp and Gln. Also preferably the non-polar residues of said region of alternating polar and non-polar amino acids are selected from the group consisting of Ile, Val, Leu, Phe, Gly, Ala and Cys, preferably selected from the group consisting of Ile, Val, Leu and Phe, and more preferably selected from the group consisting of Ile, Val and Leu. It is preferable to combine the preferred and more preferred groups of polar residues with the preferred group of non-polar residues, and more preferably with the more preferable group of non-polar residues.

In another preferred embodiment at least two - for example 2, 3, 4, 5, 6, 7 or 8 - of the polar residues of said region of alternating polar and non-polar amino acids are charged amino acid residues. As used herein charged amino acid residues are the positively charged amino acids histidine, leucine and arginine and the negatively charged amino acid residues aspartate, glutamate but also serine, threonine and tyrosine, which can be in a phosphorylated state within the cell and are then negatively charged. Preferably at least one
- for example 1, 2, 3 or 4 - of the polar residues of said region of alternating polar and non-polar amino acids is a histidine residue, a lysine residue or an arginine residue, more preferably a lysine residue or an arginine residue, while at the same time at least one other
- for example 1, 2, 3 or 4 - amino acid residue of the polar residues of said region of alternating polar and non-polar amino acids is a serine, threonine, tyrosine, aspartate or glutamate residue, preferably a serine, aspartate or glutamate residue, more preferably an aspartate or a glutamate residue. Having the same number of negatively and positively charged amino acid residues in said polar/non-polar region, i.e. one positively charged amino acid residue and one negatively charged amino acid residue or two negatively charged amino acid residues and two positively charged amino acid residues, or three negatively charged amino acid residues and three positively charged amino acid residues, can help in the formation of aggregates.

In a preferred embodiment, the region of alternating polar and non-polar amino acid residues is at a defined position with respect to a flexible linker region, for example starting 17-35 amino acids upstream (N-terminally) of such a flexible linker region. Preferably said region starts 23-31 amino acids upstream of said flexible linker region, more preferably 24-30 amino acids upstream, even more preferably 25-29 amino acids upstream and most preferably 26-28 amino acids upstream of said flexible linker region, always counting from the first amino acid of the flexible linker region.

In another preferred embodiment the polypeptide of the invention comprises a microtubule-binding domain of a tau protein, preferably a mammalian tau protein, and more preferably a human tau protein, wherein a cysteine residue is located between 17 and 30 amino acid residues upstream (N-terminally) of said flexible link region, preferably 8-12 amino acid residues upstream, more preferably 9-11 amino acid residues upstream and most preferably 10 amino acid residues upstream of said flexible linker region. The presence of cysteine residues in the MBDs of the polypeptide of the invention may help in aggregate formation. Preferably, the polypeptide of the invention comprises an even number, like e.g 2 or 4, MBDs, with one cysteine residue at the indicated position relatively to the flexible linker each. A cysteine residue may be present at said position in an unmodified microtubule-binding domain derived from a naturally occurring tau protein, or it may be present in a modified microtubule-binding domain. The presence of cysteine residues at the indicated position relative to the flexible linker may aid in the correct alignment of the microtubule-binding domains during the formation of aggregates and may therefore increase the overall rate or extent of aggregate formation of the polypeptide of the invention.

In another preferred embodiment the polypeptide of the invention comprises such modified microtubule-binding domains which differ in only 2-5 amino acid residues, and preferably in only 3-4 amino acid residues from any one of the polypeptides specified as SEQ ID NO's 1-16, more preferably from any one of the polypeptides specified as SEQ ID NO's 1-4.
In another preferred embodiment the polypeptide of the invention comprises such a modified microtubule-binding domain which differs only minimally from any one of the polypeptides specified as SEQ ID No's 1-16 and preferably SEQ ID NO's 1-4, outside of said region(s) of alternating polar and non-polar amino acid residues, that is the amino acid changes in the modified microtubule-binding domain outside of said polar/non-polar region are only up to three, preferably up to two, more preferably up to one changes of amino acid residues from the wildtype sequence. It is preferred that the changed amino acid residues are semi conservative or conservative amino acid residue changes, more preferably conservative amino acid residue changes. It is also preferred that proline residues are not introduced into the polypeptide sequence of the modified microtubule-binding domain by such a change. Most preferably, the sequences outside of said polar/non-polar region are identical to any one of the polypeptide sequences SEQ ID NO's 1-4.

In another preferred embodiment a polypeptide of the invention can comprise 2, 3, 4 or more microtubule-binding domains, some of which are modified microtubule-binding domains, but it is preferred that the polypeptide of the invention contains overall 2, 3, 4, 5, 6, 7 or 8, and preferably contains 2, 3 or 4 microtubule-binding domains. Additional polypeptide sequences may be present, but preferably no additional polypeptide sequences of microtubule-binding domains or modified microtubule-binding domains.

In another preferred embodiment the polypeptide of the invention comprises 2 or more, and preferably 2, 3 or 4 modified microtubule-binding domains, and more preferably it contains 2, 3 or 4 modified MBDs. Additional polypeptide sequences may be present, but preferably no further polypeptide sequences of microtubule-binding domains or modified microtubule-binding domains.

In a further preferred embodiment the polypeptide of the invention, when comprising at least two - for example 2, 3, 4, 5, 6, 7 or 8 - modified microtubule-binding domains, has the regions of alternating polar and non-polar amino acid residues at about the same relative position within the MBDs, that is when each of the two regions of alternating polar and non-polar amino acid residues is compared with regard to its start site relative to a flexible linker region, said polar/non-polar region starts at the same number of amino acid residues upstream of a flexible linker region, allowing a difference of 6, 5, 4, 3 or 2 amino acid residues and preferably only 1 amino acid residue, but most preferably said regions of alternating polar and non-polar amino acid residues start at the same number of amino acid residues upstream of said flexible linker regions. This arrangement puts the regions of alternating polar and non-polar amino acid residues in register with one another and can aid in increasing the rate or the extent of aggregate formation of the polypeptide of the invention.

In a further preferred embodiment the polypeptide of the invention further comprises a polypeptide sequence having at least 90%, preferably 95%, more preferably 98% and most preferably 100% identity with any projection domain of a mammalian tau protein, an particularly with any one of the polypeptide sequences listed as SEQ ID NO's 20-24. Those polypeptide sequences are the polypeptide sequences of naturally occurring projection domains of human tau proteins. Preferably, said polypeptide sequence with a high degree of identity to a projection domain is located N-terminally from a modified microtubule-binding domain ( in particular in case of homology to SEQ ID NO's 20-23). More preferably, said polypeptide with high degree to naturally occurring projection domain is located N-terminally from all microtubule-binding domains occurring in the polypeptide of the invention. Such polypeptides are preferred which contain only one polypeptide with a degree of identity to a projection domain N-terminally to 3 or 4 microtubule-binding domains, of which 1, 2, 3 or 4 can be modified microtubule-binding domains. In another preferred embodiment the projection domain SEQ ID NO.:24 may be located C-terminally to the modified microtubule binding domain(s).

In a preferred embodiment the polypeptide of the invention is a full length tau polypeptide wherein the polypeptide sequence corresponding to amino acids 275-286, 306-317 or 337-349 of human tau 441 comprises at least 7 alternating polar and non-polar amino acids, and preferably contains 7, 8, 9, 10 and more preferably 11 alternating polar and non-polar amino acids, or wherein the polypeptide sequences corresponding to amino acids 275-286 and 306-317; 275-286 and 337-348; or 306-317 and 337-348 each comprise at least 6 alternating polar and non-polar amino acids, preferably wherein the polypeptide sequences corresponding to amino acids 276-286 and 306-317; 275-286 and 337-348; or 306-317 and 332-343 each contain 7, 8, 9, 10 and more preferably 11 alternating polar and non-polar amino acids; or wherein the polypeptide sequences corresponding to amino acids 275-286 and 306-317 and 337-348 each comprise at least 5 alternating polar and non-polar amino acids, preferably wherein the polypeptide sequences corresponding to amino acids 275-286 and 306-317 and 337-348 each contain 6, 7, 8, 9, 10 and more preferably 11 alternating polar and non-polar amino acids. With regard to the nature of preferred alternating polar and non-polar amino acid residues, reference is made to the description of the preferred combinations of polar and non-polar amino acids on pages 10 and 11.

In a preferred embodiment the region of alternating polar and non-polar amino acids starts with a polar amino acid.

In a further preferred embodiment the polypeptide of the invention comprises a polypeptide having a sequence of SEQ ID NOs 17, 18 or 19. In a particularly preferred embodiment the polypeptide is any one of the polypeptides listed as SEQ ID NO. 34, 35, 36 or 37, with SEQ ID NO. 37 being the most highly preferred polypeptide.

In a further preferred embodiment the polypeptide of the invention further comprises a peptide/polypeptide sequence for purification and/or detection purposes. Such purification and/or detection "tags" are well known in the art and comprise multi-histidine tags like a hexa-histidine tag (purification/detection), the HA-tag, a calmodulin-binding domain (detection/purification), or the tags described in EP 1 231 276 (purification/detection), to name but a few.

The present invention relates also to a nucleic acid molecule which comprises a nucleic acid sequence which encodes a polypeptide of the invention.

In a further embodiment this nucleic acid molecule takes the form of an expression cassette which comprises said nucleic acid molecule, but preferably also further comprising expression control sequences which are operatively linked to said nucleic acid molecule encoding a polypeptide of the invention. The expression control sequences are chosen so that they allow expression of the polypeptide of the invention in a host. For example a nucleic acid sequence encoding a polypeptide of the invention can be isolated and cloned into an expression vector and the vector can then be transformed into a suitable host cell for expression of the polypeptide of the invention. Such a vector can be a plasmid, a phagemid or a cosmid. For example, a nucleic acid molecule of the invention can be cloned in a suitable fashion into prokaryotic or eukaryotic expression vectors, preferably into eukaryotic expression vectors and more preferably into expression vectors allowing expression in a mammalian and in particular in a human cell (*Molecular Cloning: A Laboratory Manual,* 3rd edition, Eds. Sambrook et al., CSHL Press 2001). Such expression vectors typically comprise at least one promoter and can also comprise a signal for translation initiation of the reading frame encoding the polypeptide of the invention and - in the case of prokaryotic expression vectors ― a signal for translation termination, while in the case of eukaryotic expression vectors the expression cassette preferably comprises expression signals for transcriptional termination and polyadenylation. Examples of suitable eukaryotic expression vectors are those described by Ficcerone *et al., "Generation of recombinant Baculovirus DNA in E. coli using Baculovirus shuttle vector"* (1997) volume 13, U. Reischt Eds. (Totoba NJ: Humana Press Inc.) for the expression in insect cells via baculovirus vectors, and for expression in mammalian cells, e.g. the SV40 or CMV vectors, which are commonly known and commercially available, or the sindbisvirus expression system (Schlesinger (1993), *Trans. Bio. Technol*. 11(1):18-22) or an adenovirus expression system (Hea *et al*. (1998), *Proc. Natl. Acad. Sci. USA* 95:2509-2514), or a Semliki Forest Virus-based expression system (Smerdou and Liljestrom, 1999) or a lentivirus-based expression system (Ehrengruber, M.U., and Lundstrom, K. (2000). Alphavirus-mediated gene transfer in neurons. In *Current Protocols in Neuroscience,* eds. Crawley, J., Gerfen, C., McKay, R., Rogawski, M., Sibley, D. and Skolnik, P. John Wiley & Sons, New York, Unit 4.21; Smerdou and Liljestrom (1999) *J Virol*. 73(2):1092-8; Naldini *et al*. (1966) *Science*. 1966 Apr. 12;272(5259):263-7). The molecular biological methods for the production of these expression vectors are well known to the skilled person, as well as the methods for transfecting host cells and culturing such transfected host cells. In a preferred embodiment the above-mentioned expression control sequences specify induced expression of the polypeptide of the invention, that is induced transcription of the messenger RNA encoding the polypeptide of the invention upon addition or withdrawal of an external signal, such as a small chemical like tetracycline or a hormone like Ecdysone, but the extracellular signal can also be an increase or decrease in temperature or ionizing radiation. Also, inducible expression can be brought about by inducible translation initiation of the messenger RNA or a system in which mRNA stability is controlled in an inducible fashion. Examples of expression control sequences allowing induction of polypeptide production are reviewed in the following publications: the TET-off/TET-on system, suitable for both cell cultures and transgenic animals, for example described by Gossen and Bujard (1992) *Proc Natl Acad Sci USA,* 15;89(12):5547-51; or by Gossen *et al*. (1995) *Science,* Jun 23;268(5218):1766-9; adapted for use in transgenic animals by, *for example,* Furth *et al*.(1994) *Proc Natl Acad Sci USA.* 91:9302-9306; or by Kistner *et al*. (1996) *Proc Natl Acad Sci USA* 93:10933-10938, but also the expression control system based on Cre-recombinase based methods, predominantly for use in transgenic animals, for example described by Lakso *et al*. (1992) *Proc Natl Acad Sci USA*. Jul 15;89(14):6232-6; or by Orban *et al*. (1992) *Proc Natl Acad Sci USA,* Aug 1;89(15):6861-5. A further inducible expression system, for use in both cell culture and transgenic animals is based on the insect hormone Ecdysone, for example described by Hoppe *et al*. (2000) *Mol Ther,* 1:159-164; or by No *et al*. (1996) *Proc Natl Acad Sci USA,* 93 :3346-3351. Another inducible expression system is the GAL4 system, which has been successfully applied with mice (Ornitz *et al*. (1991) *Proc Natl Acad Sci USA,* Feb 1;88(3):698-702), zebrafish (Scheer *et al*. (1999) *Mech Dev.,* Feb;80(2):153-8) and Drosophila (Brand and Perrimon (1993) *Development,* Jun;118(2):401-15), and allows conditional expression at 26-29 degrees, or also a Rapamycin based conditionals expression system (Ho *et al*. (1996) *Nature,* Aug 29;382(6594):822-6; and Pollock *et al*. (2000) *Proc Natl Acad Sci USA,* Nov 21;97(24):13221-6). A temperature-sensitive expression system is based on a Sindbis virus expression cassette (Boorsma *et al*. (2000) *Nat Biotechnol,* Apr;18(4):429-32) and predominantly suitable for controlled expression in cell culture systems.

In another aspect this invention relates to a host cell comprising a polypeptide of the invention and/or a nucleic acid of the invention and/or an expression cassette of the invention. Such a host cell can be a mammalian, non-human cell inside or outside of the animal body or a human cell outside of the human body. But it can also be an insect cell, like a drosophila cell, in culture or in the context of a transgenic insect, like a transgenic *Drosophila.* It can also be a nematode cell, like, for example, present in transgenic C. *elegans.* Preferred host cells are mammalian, and particularly human, neuronal cells, microglia cells, astrocytes, oligodendrocytes, fibroblasts, monocytes, and macrophages and other non-neuronal primary cells, which can be kept in primary tissue culture and can be made capable of expressing the polypeptide of the invention by introducing the nucleic acid/or the expression cassette of the invention, for example by transfection with such a nucleic acid or expression cassette. Other means of introducing the nucleic acid and/or the expression cassette of the invention to the above-mentioned primary cells are "gene gun" approaches, mRNA transfer, viral infection, microinjection or liposomal nucleic acid transfer, to name but a few. Other suitable host cells are mammalian cells like HEK cells, HELA cells, PC12 cells, CHO cells, JURKAT cells, mouse 3T3 fibroblasts, mouse hepatoma cells, human neuroblastoma cells, but also established cancer cell lines, particularly neuronal cell lines, of mammalian and particularly of human origin available from ATCC (www.atcc.org). The nucleic acid and/or the expression cassette of the invention can also be introduced into those cells by the above-mentioned nucleic acid transfer methods. Particularly preferred are stably transformed cell lines wherein the expression of the polypeptide of the invention is inducible. Since expression of the polypeptide of the invention shows increased neurotoxicity and/or cell toxicity when compared with the same level of expression of the wild-type human tau protein, it is preferred that in such host cells the expression of the polypeptide of the invention is usually very low or off, for example during the generation of a stably transformed cell line, and only for experimental purposes and after establishment of such a stably transformed cell line the expression of the polypeptide of the invention is turned on by addition of a suitable stimulus, like e.g. a hormone like Ecdysone or a small chemical like the antibiotic tetracycline. Again, the above described examples of expression control sequences allowing induction of polypeptide production are suitable for this purpose, like the TET-off/TET-on system, the Cre-recombinase based methods, the Ecdysone system, the GAL4 system, Rapamycin-based systems,or the above described temperature-sensitive expression system based on a Sindbis virus expression cassette.

In another aspect the invention relates to transgenic animals which comprise a host cell of the invention. Particularly preferred are transgenic flies, like transgenic *Drosophila,* transgenic nematodes, like transgenic *C*. *elegans,* transgenic fish, like transgenic zebra fish, and transgenic non-human mammals, like transgenic rodents (mice, rats). For the generation of transgenic mice with suitable cell- or tissue-specific promoters, reference is made to Hogan D. et al. (1994) "Production of transgenic mice" in *Manipulating the Mouse Embryo: A Laboratory Manual* - Hogan D., Konstantini F., Lacey E. Eds., Cold Spring Harbor Laboratory Press, Cold Spring Harbor, NY, pp. 217-252. As mentioned above, expression of the polypeptide of the invention can be inducible. With regard to inducible expression systems in transgenic mice, reference is made to Albanese C. *et al*. "Recent advances in inducible expression in transgenic mice" (2002) *Semin. Cell. Div. Biol*. **13**:129-41. Methods for the generation of transgenic flies, nematodes, and zebra fish are also well established and - in addition to the references already given above - by way of example reference is made to the following documents: Rubin and Spreadling (1982) *Science* **218**:348-53, for the generation of transgenic flies; Mellow and Fire (1995) in: *Methods in Cell Biology,* Volume 48, H.P. Epstein and T.C. Shakes, Eds. (San Diego, CA Academic Press), pp. 4451-482, for the generation of transgenic worms; Higashima *et al*. (1997) *Dev. Biol*. **192**:289-99, for the transformation of zebra fish. Most of the inducible expression systems mentioned above could be used for conditional expression in animal model systems. Under the control of tissue-specific promoters expression could be targeted to the CNS in mice and others. Most tissue-specific promoters could be used, for example, also in the context of viral vectors. In the following, tissue-specific promoters of rodents are listed. Expression in astrocytes: GFAP-promoter, macrophage colony-stimulating factor (c-fms). Expression in neurons: synapsin promoter, thy-1 promoter, neuron-specific rat enolase promotor (NSE), L7 promoter (Purkinje cells), dopamine beta-hydroxylase (DBH) promoter (predominantly in the peripheral nervous system), brain dystrophin promoter, calmodulin gene II and III promoter (CaMII, CaMIII), human and murine neurofilament light gene promoter (NF-L), human hypoxanthine phosphoribosyltransferase (hHPRT) promoter, corticotropin-releasing hormone (CRH), T alpha 1 alpha-tubulin promoter, murine low-affinity NGF receptor promoter, hippocalcin gene promoter, olfactory marker protein (OMP) promoter (olfactory neurons), GABA(A) receptor alpha 6 subunit promoter, GABA(A) receptor delta subunit promoter, tyrosine hydroxylase (TH) promoter, mouse vesicular acetylcholine transporter (VAChT), mouse glutamate decarboxylase 65 and mouse glutamate decarboxylase 67 genes promoters, brain-specific promoter of the human FGF1, gonadotropin-releasing hormone (GnRH) promoter, N-methyl-D-aspartate receptor 2A subunit gene promoter; mouse metabotropic glutamate receptor subtype 6 (mGluR6) upstream sequence, Rod photoreceptor cGMP phosphodiesterase (PDE6), human blue opsin promoter and rhodopsin promoter (retina). Neuron-restrictive silencer elements : Neuron-restrictive silencer elements (NRSEs). Expression in oligodendrocytes: MBP (myelin basic protein), proteolipid protein (PLP) promoter.

The above-mentioned tissue specific promoters are described in detail in the following documents: Turnley *et al*. (1991) *Nature,* Oct 10;353(6344):566-9; Fuss *et al*. (2001) *Mol Cell Neurosci,* Aug;18(2):221-34; Tkachuk *et al*. (1997) *Neurosci Lett,* Apr 4;225(2):121-5; Morimoto *et al*. (2002) *Epub,* Mar 26;9(2):113-20; Brenner *et al*. (1994) *J Neurosci,* Mar;14(3 Pt 1):1030-7; Forss-Petter *et al*. (1990) *Neuron* Aug;5(2):187-97; Chin, *Kachinsky et al*. (1999) *Brain Res Mol Brain Res,* Apr 20;67(2):239-46 ; Andra *et al*. (1996) *Neurobiol Aging,* Mar-Apr;17(2):183-90; Oberdick *et al*. (1990) *Science* Apr 13;248(4952):223-6; Mercer *et al*. (1991) *Neuron* Nov;7(5):703-16; Kammesheidt *et al*. (1992) *Proc Natl Acad Sci USA,* Nov 15;89(22):10857-61; Matsuo *et al*. (1993) *Brain Res Mol Brain Res*, Oct;20(1-2):9-20; Leconte *et al*. (1994-95) *J Mol Neurosci*, 5(4):273-95; Chen *et al*. (1994) *Proc Natl Acad Sci USA*, Mar 29;91(7):2611-5; Rincon-Limas *et al*. (1994) *J Neurosci Res,* Jun 15;38(3):259-67; Keegan *et al*. (1994) *Mol Cell Neurosci,* Dec;5(6):505-14; Gloster *et al*. (1994) *J Neurosci,* Dec;14(12):7319-30; Carroll *et al*. (1995) *J Neurosci,* May;15(5 Pt 1):3342-56; Ueda *et al*. (1997) *J Neurosci,* May 1;17(9):3014-23; Grant *et al*. (1996) *Neuroscience* Dec;75(4):1099-115; Walters *et al*. (1996) *Int J Dev Neurosci,* Nov;14(7-8):813-22; Jones *et al*. (1996) *J Neurochem,* Sep;67(3):907-16; Shimoda et al. *(1995) Brain Res Mol Brain Res,* Jul;31(1-2):61-70; Liu *et al*. (1997) *Brain Res Mol Brain Res,* Oct 15;50(1-2):33-42; Luscher *et al*. (1997) *Brain Res Mol Brain Res,* Nov;51(1-2):197-211; Naciff *et al*. (1999) *J Neurochem,* Jan;72(1):17-28; Millecamps *et al*. (1999) *Nat Biotechnol,* Sep;17(9):865-9; Makinae *et al*. (2000) *J Neurochem,* Oct;75(4):1429-37; Chiu *et al*. (2000) *Oncogene,* Dec 14;19(54):6229-39; Taylor *et al*. (2001) *Biochem Biophys Res Commun,* Mar 30;282(2):543-7; Kim *et al*. (2002) *J Biol Chem,* Feb 15;277(7):5194-202; Desai *et al*. (2002) *J Biol Chem,* Nov 29;277(48):46374-84.

In another aspect the invention relates to a diagnostic kit for the detection of the aggregated form of human tau protein which comprises a) an antibody which specifically binds to the aggregated form of human tau protein, and b) a polypeptide of the invention or an extract obtained of a host cell expressing the polypeptide of the invention or of a transgenic animal expressing a polypeptide of the invention. A polypeptide of the invention or the host cell or transgenic animal extract serve as a positive control for the aggregated form of human tau protein in such a diagnostic kit. This is because the polypeptide of the invention readily aggregates in such a way that is immunologically equivalent to the pathogenic, aggregated form of human tau protein. Such a diagnostic kit can be useful for determining whether the pathogenic form of human tau protein has formed in a patient, and if yes, how much of the pathogenic form of the human tau protein has formed. Such a quantitative assay, i.e. a quantitative ELISA for the pathogenic form of human tau protein, is now possible having regard to the present invention because defined quantities of a polypeptide equivalent with the human tau protein in its aggregated form can now be obtained, for example by purification of the polypeptides with SEQ ID NOs:34-37, and in particular the polypeptide SEQ ID NO. 37, from transfected human SHS Y5Y neuroblastoma cells, which are shown in Example 4. Antibodies which specifically bind to the aggregated form of human tau protein are, for example, the antibody AT8 and AT100, as described in Example 4. Human tau protein in its aggregated form can be obtained by adapting the procedure of PHF isolation from AD brains (Greenberg and Davies (1990) *Proc Natl Acad Sci USA,* Aug; 87(15):5827-31). The peptide could be isolated by means of standard biochemical methods. Protein isolation can be carried out preferably 6-48 hrs after after the beginning of expression, more preferably 24-36 hrs after the beginning of expression.

In another aspect the invention relates to a polypeptide of the invention or a nucleic acid of the invention or an expression cassette of the invention or an extract of a host cell of the invention or an extract of a transgenic animal of the invention for use in medicine and/or veterinary medicine and in particular for use in a method for the generation of conformation-specific anti-tau antibodies which selectively recognize human tau in the aggregated conformation, wherein the method comprises the administration of a polypeptide of the invention or of an extract of the host cell of the invention or of an extract of the transgenic animal of the invention or of an expression construct of the invention or of a nucleic acid of the invention to an animal to induce an immune response against the aggregated conformation of a tau protein, for example a human tau protein. In the example of the amyloid-β-peptide vaccination with amyloid-β-peptide can lead to a decrease of senile amyloid plaques in a patient, probably due to an immune response against the amyloid-β-peptide, thereby lowering the patient's senile amyloid plaque burden. The generation of a immune response in a subject, e.g. a human or animal patient, against the aggregated/pathogenic form of human tau protein, can lead to the reduction of NFTs in the extracellular environment, e.g. from dying or deceased cells and may, by reducing an inflammatory response, stop the spread of the pathogenic form of tau from sites of initial tau aggregation, like the transentorhinal cortex or the hippocampus, to regions of the temporal cortex and the even further spread to regions of polymodal association cortices, and thus may prevent the onset of memory loss and dementia. Thus, vaccination with a polypeptide of the invention or an extract comprising a polypeptide of the invention may provide a new treatment of tauopathies and in particular a prophylaxis against tauopathies.

In another aspect, the invention relates to a screening method for identifying molecules which alter a phenotype induced by a polypeptide of the invention. As described in the Examples, expression of a polypeptide of the invention in a host cell induces specific phenotypes, like aggregation of the expressed polypeptide of the invention, in particular the formation of immobile aggregates as measured by FRAP, but also the induction of cell toxicity over time, for example the induction of neurotoxicity in the human neuronal cell line SHS Y5Y, as described in Example 5, and also the induction of aggregates which are immunoreactive against antibodies specific for the pathological/aggregated form of human tau like the Abs AT8 and AT100. Another induced phenotype is the induction of apoptotic cell death over time when the polypeptide of the invention is expressed. In particular, the apoptosis induced by the polypeptide of the invention is atypical in that the resulting nuclei are pyknotic, but not fragmented. An alteration, that is either a significant increase of the severity of the induced phenotype or a significant decrease of the severity of the induced phenotype, can therefore be used to identify molecules with an effect on tau pathology. Such a screening method can comprise the following steps: a) providing a host cell of the invention or a transgenic animal of the invention, in which preferably the polypeptide of the invention is induced to be expressed, more preferably only several hours before or after the start of the screening method, like 0-40 or 0-35 or 0-30 or 0-25 hours before or after the start of the screening method, b) adding a molecule to be tested (defined as start), and c) identifying the molecules the addition of which has led to a change of the induced phenotype in cell culture or in the transgenic animal. A change of the induced phenotype is then indicative of such molecules which alter the phenotype induced by the polypeptide of the invention. The molecule to be tested can be a small chemical molecule and can be added just by admixture to, for example, the cell culture medium in a well in which the host cells of the invention are cultured, or by addition to the food of the transgenic animal of the invention. Of particular interest are such molecules which, when added, for example at a concentration of 1pM-1mM, more preferably 100pM-100µM, even more preferably 1nM-10 µM, lead to an amelioration of the induced phenotype, i.e. to a decrease of the neurotoxicity of the expressed polypeptide of the invention or to a decrease of the induced tau aggregation or to a decrease of the positive signal of such antibodies, like AT100, which are specific for pathogenic tau-phosphorylation. Thus, in a preferred embodiment, the screening method provides a host cell of the invention or a transgenic animal of the invention in which expression of the polypeptide of the invention is inducible. A couple of hours after or prior to the addition of the molecule to be tested, for example 48 hours before to 48 hours after said addition, more preferably 24 hours before to 24 hours after said addition, even more preferably 12 hours before to 12 hours after said addition and most preferably 6 hours before to 6 hours after said addition, expression of the polypeptide of the invention is induced, then the molecule to be tested, for example the small chemical molecule to be tested, is added and then the host cells of the invention or the transgenic animals of the invention are further incubated in the presence of the molecule to be tested up to a time point at which the expression of the polypeptide of the invention normally has led to a detectable phenotype, for example in a cell in cell culture or in a transgenic worm or fly of the invention. Then the host cell of the invention and the transgenic animal of the invention to which a molecule to be tested has been added is compared with a control host cell of the invention or a control transgenic animal of the invention where no molecule to be tested had been added and where the unperturbed phenotype of aggregated-tau expression is visible for comparison. Those molecules with an effect on tau aggregation can now be identified by way of their potential to decrease neurotoxicity and/or decrease tau aggregation and/or decrease tau phosphorylation and/or decrease cell toxicity. In a preferred embodiment, such a screening method is carried out in a high throughput screening format, i.e. the host cells of the invention are, for example, grown in multiple-well dishes, like 96-well dishes, and a large number of molecules to be tested can be tested in one experiment, for example by adding one compound to be tested to one well each. A skilled person will appreciate that HTPS-screening can also be performed with transgenic animals of the invention, like for example with transgenic *C. elegans* or transgenic *Drosophila.* This method is useful to identify small chemical compounds or peptides or polypeptides of clinical interest. Those molecules can be beneficial therapeutics in the treatment of tauopathies, such as the neurodegenerative diseases mentioned above as taopathies. In a preferred embodiment the small chemical compounds or the peptides or the polypeptides identified by the above-mentioned screening method are formulated into a pharmaceutical composition which can then be used for the treatment of the above-mentioned tauopathies.

In another aspect the invention relates to a method for identifying proteins which interact with the aggregated/pathogenic form of tau. This method comprises the steps of providing a host cell of the invention or transgenic animal of the invention in which a polypeptide of the invention has preferably been induced to be expressed and identifying those polypeptides/proteins which interact with the polypeptide of the invention. Since the expression of the polypeptide of the invention may lead to cell toxicity or neurotoxicity, depending on the expression level, it is preferred that such host cells of the invention and transgenic animals of the invention are used for this method in which expression of the polypeptide of the invention is off, or at a very low basal expression level, and only induced shortly, i.e. 1-30 hours, and more preferably 2-20 hours, before the method of identifying interacting proteins is performed. In a preferred embodiment expression of the polypeptide of the invention is induced, for example by ecdysone or by tetracycline addition or by the other Inducible systems described above, and then after the indicated time span the host cell of the invention or the transgenic animal of the invention is harvested and processed for biochemical purification of the polypeptide of the invention. Biochemical purification methods of the aggregated form of tau protein are described in Greenberg SG and Davies P, cited above. Alternatively, biochemical purification of the polypeptide of the invention can be facilitated by the presence of a peptide or a polypeptide sequence aiding in purification purposes, like immunoprecipitation or the TAP-tag described in Puig *et al*. below. The generation of cell extracts suitable for biochemical purification of the polypeptide of the invention will be clear to a skilled person having regard to the present invention. The biochemical purification of the polypeptide of the invention under conditions which preserve biomolecular complexes, that is protein-protein and protein-nucleic acid interactions, allows the subsequent identification of polypeptides/proteins, but also of e.g. RNAs, which interact with the aggregated form of tau because such binding polypeptides/proteins will be in the purified fraction together with the polypeptide of the invention, where they can then be detected by immunological, biochemical and/or biophysical methods and in particular by mass-spectroscopical methods. The biochemical method can be the analysis of the purified fraction on an SDS gel and the subequent determination of the polypeptide sequence of co-purifying bands, for example by the well-known Edman-degradation. However, co-purifying bands can also be readily partially sequenced using mass-spectroscopical methods, like for example the method described by Wilm et al. (Mann and Wilm (1994) *Anal Chem,* Dec 15;66(24):4390-9; Shevchenko *et al*. (1996) *Proc Natl Acad Sci USA,* Dec 10;93(25):14440-5.)
Alternatively, the polypeptides/proteins interacting with the aggregated form of tau, are identified by protein-protein interaction assays like two-hybrid assays or microscopy-based protein-protein interaction assays, preferably FRET or FRAP. Two-hybrid assays detect the physical interaction between two or more molecules of interest when the act of association between the interactive partners leads to production of a readily observed biological or physical readout. The classical yeast two-hybrid system detects the interaction between X and Y by the induction of transcription of easily scored responsive reporters that is brought about by the interaction of the translational fusion of X with a DNA-binding domain on the one hand with the translational fusion of Y fused to a transcriptional activation domain on the other. However, other two-hybrid approaches based on the artificial production of two-hybridic molecules, but varying in the generation of the readout from induction of transcription, repression of transcription, activation of signal transduction pathways or reconstitution of a disrupted enzymatic activity are possible. For review of two-hybrid-based protein-protein interactions we refer to Toby and Golemis (2001) *Methods* 24(3):201-17. In the method if the invention a polypeptide of unknown function (Y), or a polypeptide with a suspected involvement in tauopathies (Y), is fused with a fluorescent polypeptide, like GFP (the fusion is further called Y-GFP), and expressed in cells which also express a polypeptide of the invention, like the M123 mutant, and as a control in cells without mutant tau. The mobility of the Y-GFP can now be determined with microscopy-based methods, like FRAP. If the mobility of Y-GFP is reduced in the cells expressing the polypeptide of the invention, but not in the control cells, it is likely that the polypeptide Y interacts with the tau aggregates. In a preferred embodiment, the polypeptide of the invention in this method is fused to a second fluorescent polypeptide, which is spectrally separated from the fusion partner of the polypeptide of interest Y. Preferably the two fluorescent polypeptides form a FRET donor-acceptor pair. Now both, the relative mobility of Y and of the polypeptide of the invention can both be determined. If the mobility of Y is reduced at positions in the cell that are also occupied by the tau-aggregate, Y is said to have an affinity for the tau aggregate. For this, microscopy-based assays may be used. They are preferably based on FRET and/or FRAP, as explained below.
FRET (Foerster or Fluorescence Resonance Energy Transfer) is a spectroscopic technique that can be exploited in microscopy and reports on the separation distance between two fluorophores with extreme distance dependence (Bastiaens PI, Squire A. Fluorescence lifetime imaging microscopy: spatial resolution of biochemical processes in the cell. *Trends Cell Biol*. 1999 Feb;9(2):48-52 (review); Wouters et al (2001) "Imaging biochemistry inside cells." *Trends Cell Biol,* May;11(5):203-11.)

In FRET, the energy stored in the excited state of a fluorophore (the donor) upon absorption of a photon, is transferred non-radiatively to a second fluorophore (or chromophore), the acceptor. This transfer is due to dipole-dipole interactions between the emission dipole of the donor and the absorption dipole of the acceptor and depends on the separation distance, the orientation between the dipoles, and the extent of overlapping energy levels (the overlap integral). The inverse sixth order dependence of FRET on separation distance produces an extremely steep decline of the FRET efficiency over a couple of nanometers. Furthermore, the typical distance for most pairs at which 50% of the molecules engage in FRET (the Foerster or R0 distance) lies in the order of magnitude of average protein diameter (∼4-6 nm), giving rise to detectable FRET at a maximum distance of about 10 nm.

For the latter reason, FRET is a very popular method to assess (fluorescently labeled) protein-protein interactions and protein conformational changes. The availability of genetically expressible fluorophores, the green fluorescent proteins (GFPs), which come in many colors, allows FRET measurements in living cells.

A number of techniques are available at present to detect and quantify the occurrence of FRET. These fall into two categories: 1) Spectral, i.e. fluorescence emission intensity-based methods that are based on the loss of donor emission and concomitant gain of acceptor emission. These are: sensitized acceptor emission, ratio imaging, acceptor photobleaching-induced donor unquenching, and anisotropy microscopy. 2) Fluorescence decay kinetics-based methods that are based on the reduced donor photobleaching phenomenon and reduced donor fluorescence lifetime (or duration of the excited state) in the presence of FRET. These are: donor photobleaching kinetics and fluorescence lifetime imaging microscopy (FLIM). The latter technique is especially useful for FRET as the fluorescence lifetime is relatively independent of trivial non-FRET related events and is furthermore independent of fluorophore concentration and light path, both of which are difficult to control in a microscope. Different, functionally equivalent implementations of FLIM exist (Szmacinski *et al*. (1994) "Fluorescence lifetime imaging microscopy: homodyne technique using high-speed gated image intensifier." *Methods Enzymol,* 240:723-48; Wang *et al*. (1992) *Crit Rev Anal Chem,* 23 (5): 369-395; Clegg *et al*. (2003) *Methods Enzymol,* 360:509-42; Theodorus *et* al (1993) *Biophys. Chem.* Volume 48, Issue 2, Dec.1993, pp. 221-239.)

FRAP (Fluorescence recovery after photobleaching) (Reits and Neefjes (2001) *Nat Cell Biol,* Jun;3(6):E145-7) is a technique that reports on diffusion of fluorescently labeled biomolecules in living cells. In this technique, a high-power laser beam is used to photodestruct labeled biomolecules in a defined area of the cell. Diffusion (and transport) of molecules from neighboring non-illuminated areas can then repopulate the illuminated area, leading to a time-dependent recovery of fluorescence in this area. For the recovery kinetics, the diffusional recovery can be determined. In another implementation called fluorescence loss in photobleaching (FLIP), the high-power laser illuminates the same area in the cell for a longer period. Diffusionally connected areas in the cell, outside of the illuminated area will loose total fluorescence intensity due to continuous delivery and photodestruction in the illuminated area.

A recent implementation called fluorescence localisation after photobleaching (FLAP) (Dunn *et al*. (2002) *J Microsc,* Jan;205(Pt 1):109-12) uses two spectrally separated fluorescently labeled forms of the same biomolecule where only one of the fluorescent species is photodestructed, either in the short-term FRAP or long-term FLIP mode. The non-destructed fluorescent species now acts as reference and diffusion/transport of the biomolecule can be assessed by simple division of the two fluorescent emission bands. The loss of the photodestructed species leads to a detectable change in the image ratio and provides, in addition to diffusional velocity parameters, information on the directionality of the diffusion/transport process. The same effect can be achieved when using photoconvertible or photoactivatable fluorescent protein tags. (Zhang *et al*. (2002) *Nat Rev Mol Cell Biol,* Dec;3(12):906-18.)

The above-mentioned techniques can be used to screen for proteins (or other molecules) that bind to the tau fibrils.

By labeling the tau molecules with an appropriate FRET acceptor dye, interacting protein candidates can be detected by fusing them to an appropriate FRET donor dye and performing a FRET-based interaction assay (interaction screen). A useful implementation of a high throughput screen would be to produce a donor-labeled cDNA expression bank using a viral delivery system. Monoclonal virus particles can then be produced by limiting titration of the expression construct into multi-well packaging helper cells from which large amounts of virus particles can be harvested. Such an approach lends itself well for robotization. Either of the FRET detection techniques discussed above can be used for the analysis of interaction. Some of these techniques can also be used on fixed cells, enabling the possibility to generate an archival collection of cells that can be screened and re-screened at any given time. The FLIM technique is particularly useful for sensitive detection of interactions in the correct physiological context of the living cells.

Alternatively, FRAP/FLIP/FLAP-based screening can be used to detect tau fibril-interacting candidates. This possibility is given by the extreme immobility of the tau aggregates in living cells. By labeling the tau molecules with a particular fluorophore and the generation of a cDNA expression bank as discussed above, labeled with a spectrally separate fluorophore, the motility of the proteins in the expression bank can be directly compared with the aggregated tau. Photodestruction of the fluorophore conjugated to the interaction partner candidate and subsequent repopulation of the illuminated area provides a means of determining diffusional/transport velocities. In principle, the FLAP technique can provide the same information and in fact, taking the tau aggregates as a reference, any FLAP/FLIP measurement is already a FLIP-like determination in itself. If a protein exhibits binding affinity to the tau fibril, a detectable reduction in its motility should be apparent where the fluorescent signals of the interaction candidate and the aggregate overlap (immobilization screen). This technique can even provide information on relative binding affinities between different interaction candidates, and, in contrast to the FRET detection scheme, would also work if the interaction candidate binds to the tau aggregate indirectly through a second (or multiple) protein(s), which would raise the separation distance beyond the detection limit of FRET.

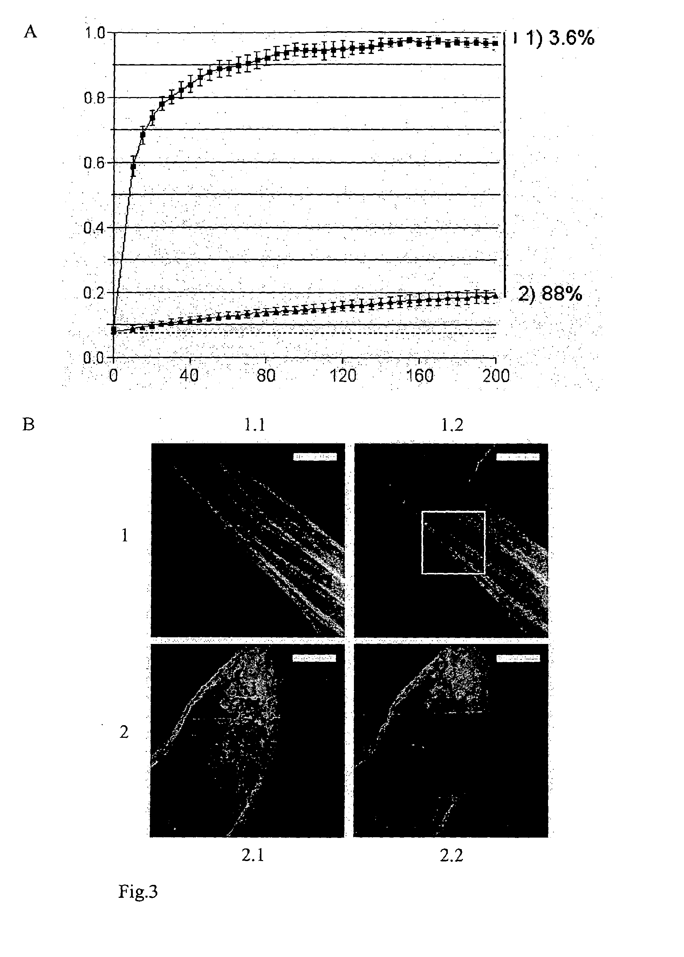

In an alternative embodiment, the polypeptides of the invention can used as the basis for a new kind of two-hybrid assay. As explained above, two-hybrid assays detect the physical interaction between two or more molecules of interest when the act of association between the interactive partners leads to production of a readily observed biological or physical readout. The classical yeast two-hybrid system detects the interaction between X and Y by the induction of transcription of easily scored responsive reporters that is brought about by the interaction of the translational fusion of X with a DNA-binding domain on the one hand with the translational fusion of Y fused to a transcriptional activation domain on the other. In the new two-hybrid assay of the invention, the polypeptide of the invention, and preferably the minimal part of the polypeptide of the invention which still mediates aggregation, i.e. the modified tubulin binding repeats of an aggregation domain of tau, like the modified tubulin binding repeats of the aggregation domain of the triple mutant M123, is fused translationally with a polypeptide X - a polypeptide for which one desires to identify interaction partners or for which one desires to confirm the interaction with another polypeptide. The new two-hybrid assay of the invention now makes use of the immobility of the polypeptides of the invention after aggregation into fibrils (see, for example, Figure 3). If a polypeptide of the invention is fused with a polypeptide X, for which one desires to find an interaction partner, this fusion polypeptide will form immobile fibrils when tranfected into a cell, for example a mammalian cell and preferably a non-neuronal cell. A polypeptide, which interacts with the fusion partner of the polypeptide of the invention, will be rendered less mobile because of this interaction. This property can be used for an interaction screen.
Cells are transfected with, for example, the M123(aggregation domain)-X fusion polypeptide and seeded into tissue culture trays with multiple wells. A second expression plasmid capable of leading to the expression of a translational fusion of a cDNA library with a fluorescent protein, like GFP (Y-GFP), in the M123-X containing cells is cotransfected or transfected shortly after seeding, for example 0-24 h. after seeding. The GFP-signal stemming from the cDNA-product-GFP (Y-GFP) polypeptide is then observed. If this signal is immobile, which can, for example, be determined by a FRAP experiment, it is likely that the proteins X and the cDNA-product protein Y interact. A construct, which leads only to the expression of the polypeptide of interest (not fused with X) serves as a control to exclude such polypeptides Y, which bind to the tau-related polypeptide sequences. Moreover, the Y-GFP polypeptides should only exhibit reduced mobility at positions within the cell, where the tau-X scaffold is located. A bona fide interaction between X and Y can be concluded when the motility of the prey Y is only affected (strongly reduced) at position within the cell where also the bait X is present in the form of an immobile scaffold. Thus, the use of a polypeptide comprising a polypeptide of the invention and a second polypeptide, for which one desires to find an interaction partner, in a protein-protein-interaction screen and/or assay, is also an aspect of the present invention.

In all protein-protein-interaction assays, microscopy-based protein-protein interaction assays may also be used and can, in fact, be preferred. They are preferably based on FRET and/or FRAP, two methods explained above.

In a preferred embodiment the method for identifying proteins interacting with the aggregated/pathogenic form of tau further comprises the identification of polypeptides/proteins which interact with the wild-type human tau protein as a negative control step. Since the wild-type human tau protein is predominantly in the non-pathogenic/non-aggregated conformation, those proteins which selectively interact with the polypeptide of the invention, but not the wild-type human tau protein, can be identified by comparing the set of interacting polypeptides/proteins for the polypeptide of the invention with the set of interacting polypeptides/proteins with wild-type human tau protein, for example in the above-mentioned two-hybrid assays or microscopy-based protein-protein interaction assays. Those proteins which selectively interact with the aggregated/pathogenic form of tau are of particular interest, as they might form disease-specific contacts with tau and might therefore constitute novel targets for the treatment of tauopathies.

In another aspect the present invention relates to a method for the generation of conformation-specific anti-tau antibodies which selectively recognize the human tau protein in the aggregated/pathogenic conformation. This method comprises the administration of a polypeptide of the invention or of an extract of a host cell of the invention which comprises expressed polypeptide of the invention or of an extract of a transgenic animal of the invention which comprises a polypeptide of the invention to an animal to induce an immune response against the aggregated form of the polypeptide of the invention. Since the aggregated form of the polypeptide of the invention takes the conformation of aggregated/pathogenic human tau polypeptide, such antibodies are induced in the immunized animal which recognize the aggregated/pathogenic conformation of human tau. This method becomes possible because the polypeptides of the invention, contrary to wild-type human tau protein, readily form aggregates taking a conformation very similar to the aggregated/pathogenic form of human tau, which up to now was not easy to obtain, and if so only in limited amounts, for example only from brain tissue of a diseased patient suffering from a tauopathy.

In a preferred embodiment this method is used for the generation of polyclonal, and particularly of monoclonal and/or recombinant antibodies, such as scFvs, dsFvs or humanized antibodies. For example, mice can be immunized with the polypeptide of the invention, after a while their lymphocytes can be isolated, for example from spleen, and fused with myeloma cells following the established procedures of monoclonal antibody generation (Harlow E., and Lane E. (1988) *Antibodies: A Laboratory Manual*, Cold Spring Harbor Laboratory, Chapter 6, *Monoclonal Antibodies,* and Chapter 7, *Growing Hybridomas*.). ScFvs and dsFvs can readily be obtained by using phage display technology in a screen for binders of the polypeptides of the invention.

In a preferred embodiment a wild-type tau protein, like the human wild-type tau protein, is used in a negative selection step to only select for such antibodies which detect the aggregated/pathogenic conformation of the tau protein, but not the wild-type tau protein in its non-aggregated conformation. Applying this to monoclonal antibody generation, only such clonal colonies are kept which interact with the polypeptide of the invention, being representative of the aggregated/pathogenic conformation, but not with wild-type tau protein, and preferably not with human wild-type tau protein.

In another aspect this invention relates to the use of a polypeptide of the invention for the preparation of a composition for immunizing a subject. It will be apparent to the skilled person that also extracts of a host cell of the invention expressing the polypeptide of the invention or extracts of a transgenic animal of the invention expressing the polypeptide of the invention can be used therefore, preferably purified fractions thereof which contain the polypeptide of the invention. The preparation of a composition for immunizing a subject, that is the preparation of a vaccine, in which this composition contains polypeptide sequences as the active component against which the immune response is elicited, is known in the art, as for example shown by U.S. Pat. Nos. 4,608,251; 4,599,231; and 4,578,770. Such vaccines are typically prepared in an injectable form, i.e. either as liquid solutions or suspensions. Solid forms which are suitable for later solution in liquids prior to injection may also be prepared. Emulsifying agents may be added to the solution. Pharmaceutically acceptable excipients which are compatible with the active immunogenic ingredient, i.e. the polypeptide of the invention in this case, are often added to said active immunogenic ingredient. Such suitable excipients are, by way of example, water, ethanol, glycerol, dextrose, saline, or the like, and combinations thereof. If desired, the vaccine may further contain small amounts of other auxiliary substances, for example wetting or emulsifying agents, pH buffers, or adjuvants which can enhance the effectiveness of the vaccine. Adjuvants can enhance the immune response against the polypeptide of the vaccine and methods of achieving such an adjuvant effect for the vaccine are known, for example, as detailed in *"The theory and practical application of adjuvants",* 1995, Duncan E.S., Stewart-Tull (Eds.), John Wiley & Sons Ltd. For use in the vaccines of the present invention such adjuvants are preferred which can facilitate overcoming the autotolerance to autoantigens. Examples of such suitable adjuvants are an oil formulation, a polymer, a micelle-forming adjuvant, aluminium adjuvants or an immune targeting or an immune modulating adjuvant. In such a vaccine the polypeptide of the invention can be present in a suitable dose range, typically of the order of several hundred micrograms per vaccination. A range from 0.1 µg - 10 mg can be suitable, preferably a range from about 0.5 µg - 1 mg, and more preferably a range from 10 µg - 250 µg. Vaccines can be administered by conventional routes, such as parenteral administration, for example by subcutaneous, intracutaneous, intradermal, subdermal or intramuscular injection. The vaccines may also be formulated in a way suitable for other modes of administration, like suitable for suppositories or even formulations suitable for oral, sublingual, intraperitoneal, epidural, spinal or intracranial administration. The above-mentioned concentration ranges are given for administration to a grown-up human subject, and it will be appreciated by the skilled person that for the preparation of a composition for immunizing an animal subject, such as a rodent or another mammal, the overall amount of the polypeptide to be administered per vaccination should be adjusted to account for the characteristics of the other subject, for example the difference in weight. Vaccinational effects can also be achieved by injection of naked DNA of the invention intramuscularly, intradermally, subcutaneously or in a similar way (Waismann et al. (1996) "Suppressive vaccination with DNA encoding a variable region gene of the T-cell receptor prevents autoimmune encephalomyelitis and activates Th2 immunity." Nature Medicine 2:899).

In another aspect the invention relates to the use of the polypeptides of the invention in the above-described *in vivo* and *ex vivo,* like *in vitro,* screening methods.

In another aspect the invention relates to the use of a polypeptide of the invention or of a nucleic acid of the invention or of a expression cassette of the invention for the induction of neurofibrillary tangles in cells and preferably in cells which are kept in cell culture. As demonstrated for example in Example 2, cells in which the polypeptide of the invention is present are induced to form neurofibrillary tangles after a certain period after the polypeptide of the invention has been introduced into said cell. In cells in cell culture the formation of neurofibrillary tangles can be expected after 10-30 hours after sufficient amounts of the polypeptide of the invention have been introduced into the cell. The polypeptide of the invention can be directly transferred into the cell in which one wishes to induce neurofibrillary tangles, for example by microinjection or by liposomal transfer of the polypeptide of the invention, but it can also be introduced into said cell by transfection of an expressable form of the nucleic acid of the invention, e.g. DNA or RNA, like mRNA, or of an expression cassette of the invention, for example by typical transfection procedures or also by microinjection. Most preferably, the production of the polypeptide of the invention inside a cell is induced by using an inducible expression system for the expression of the polypeptide of the invention, for example in a stable cell line or a transgenic animal which carry an inducible expression cassette of the invention, like the inducible systems described in detail above. The polypeptide of interest could also be expressed by means of a temperature-sensitive expression system based on Sindbis virus expression cassettes, described by Boorsma *et al*. (2000) *Nat Biotechnol,* Apr;18(4):429-32.). Specific antibodies against the polypeptide of the invention could be used for passive immunization. These specific immunoglobulins can be applied preferably by parenteral route, though a direct application into cerebrospinal fluid may also be possible.

In another aspect the present invention relates to the use of a polypeptide of the invention for the isolation of polypeptide/proteins which interact with neurofibrillary tangles. As described above, it is preferred to use such a form of the polypeptide of the invention which further carries a tag to facilitate purification under conditions which leave protein complexes intact, for example the tags and the purification conditions as described by Puig *et al*. (2001) *Methods* 24(3):218-29. As described above, the polypeptide of the invention can form neurofibrillary tangles when introduced into a cell and biochemical purification of the neurofibrillary tangles, preferably using, for example, a TAP-tagged version of the polypeptide of the invention and the subsequent biochemical analysis of the constituents of the purified neurofibrillary tangles can lead to the identification of proteins which interact with those neurofibrillary tangles. Those proteins/polypeptides or RNAs are then interesting targets for the development of further therapeutic approaches for the treatment of tauopathies.

In another aspect the present invention relates to the use of a polypeptide of the invention for the generation of antibodies which selectively recognize the tau protein, and in particular the human tau protein, in the aggregated/pathogenic conformation. As described above, also an extract of a host cell of the invention comprising a polypeptide of the invention or an extract of a transgenic animal of the invention comprising a polypeptide of the invention, but preferably a purified fraction comprising a polypeptide of the invention thereof, can be used for this purpose, for example, in one of the above-described methods for the generation of conformation-specific anti-tau antibodies. In this use, the polypeptide of the invention can be formulated as a composition suitable for immunizing a subject as described above. However, it is also contemplated that the polypeptide of the invention, which represents the aggregated/pathogenic form of the tau protein, can be used in antibody or antibody-fragment selection schemes, as described, for example, in Pini and Bracci (2000) *Curr Protein Pept Sci,* Sep;1(2):155-69); McCafferty *et al*. (1990) *Nature,* 6;348(6301):552-4), like in phage display, for example phage display of scFv- or dsFv-type antibody fragments, in order to select for such antibodies/antibody fragments which selectively bind the polypeptide of the invention. As described above, in a preferred embodiment it is also contemplated to then use the wild-type tau protein, for example the wild-type human tau protein, in a negative selection step to identify those antibodies/antibody fragments, for example out of a library displaying that antibody or antibody fragment, which only binds the aggregated/pathogenic form of the tau protein, and in particular of the human tau protein, but not the non-aggregated wild-type tau protein, and in particular not the non-aggregated wild-type human tau protein.

### DESCRIPTION OF THE FIGURES

Fig. 1 shows phosphorylation epitopes (P1, P2 and C-terminal (upper scale)), microtubule-binding domains ((TB)R1-(TB)R4), as well as the major pathological mutations within the (TB)R1-(TB)R4 and in the C-terminal, known from inherited tauopathies (lower scale). A1, A2 - acidic domains, whose presence or absence determines 3 different tau projection domains splice forms. C - C-terminal domain. (lowest scale) : 1 - projection domain; 2 - assembly domain.

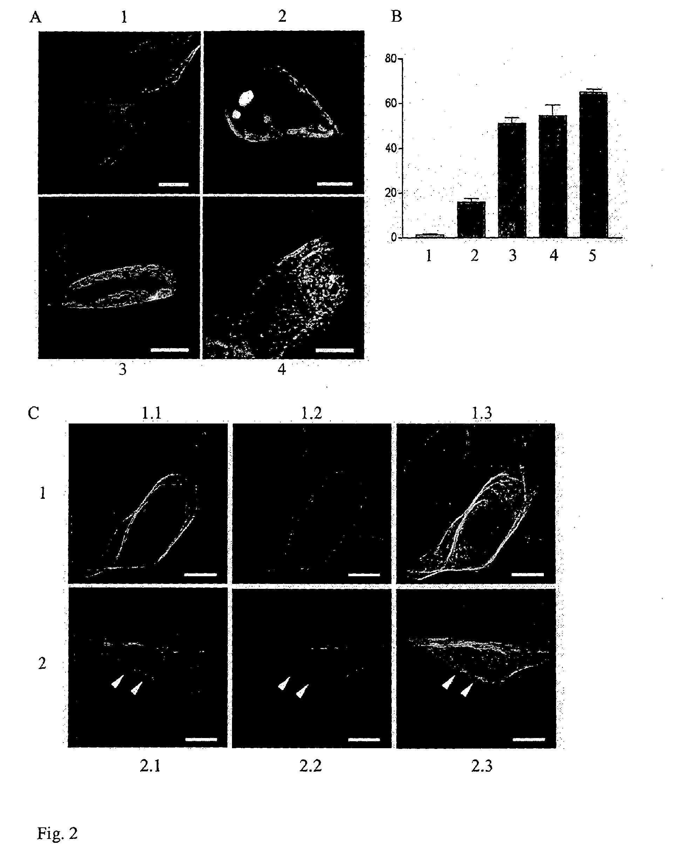

Fig. 2a) shows wild-type tau distribution in CHO cells (1) and three different types of aggregates in the triple mutant M123 (2, 3, 4). The same type of aggregates are observed also with the double mutants. Fig. 2b) shows the relative number of cells (the Y axis), possessing apparent aggregates within the cytosol. 1 - wild-type tau; 2 - M2; 3 - M23; 4 - M12; 5 - M123.
Fig. 2c). Wild-type tau transfected cells (1) showed association with microtubules (1.1 - tau-GFP, 1.2 - alpha-tubulin antibody, 1.3 - overlay, which displays yellow colour in the original colour printouts), while many aggregating mutant cells (2) showed regions of lacking overlap between the aggregates and tubulin cytoskeleton (2.1 - mutant tau-GFP, 2.2 - alpha-tubulin antibody, 2.3 - overlay, which demonstrates large regions of only red and green colour in the coloured original). All scales are 8 µm.
Fig. 3a) shows apparent differences in the FRAP-properties between the wild-type tau (1) and the triple mutant (2). While the immobile fraction of the wild-type is close to zero (3.6%), the immobile fraction of the triple mutant is 88% at 200 sec. after bleaching. The X axis shows seconds after bleaching, the Y axis - relative fluorescence recovery.
Fig. 3b) shows the fluorescence of CHO transfected cells (1 - wild-type tau; 2 - triple mutant) before (1.1 - wild-type tau before bleaching; 2.1 - triple mutant before bleaching) and 40 sec. after bleaching (1.2 - wild-type tau 40 sec. after bleaching; 2.2 - triple mutant 40 sec. after bleaching). Scale: 4 µm.

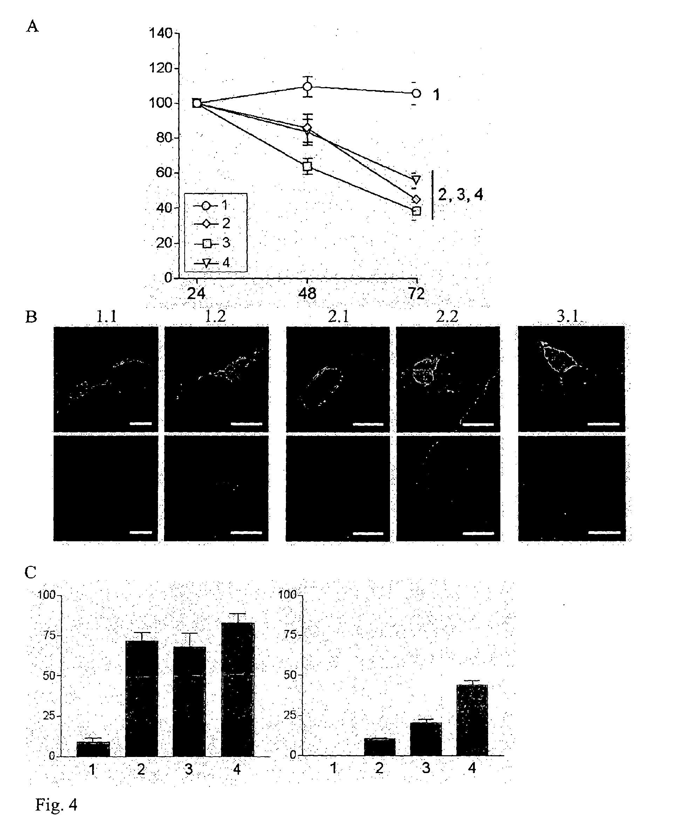

Fig. 4a) shows strong celltoxicity of double and triple mutants, already visible at 48 h. and 72 h. (1 - wild-type tau, 2 - M23, 3 - M12, 4 - M123; 1, vs. 2, 3, 4 at 72 h. p<0.001). Y-axis shows number of surviving cells as % of the starting cell number at 24h.
Fig. 4b) shows the presence of two specific phosphoepitopes after immunocytochemistry 36 h. post transfection in SHSY5Y neuroblastomas (upper row - GFP-tau constructs, lower row - specific antibody immunocytochemistry. AT8 immunolabeling: 1.1 - wild-type tau; 1.2 - M123 tau. AT100 immunolabeling: 2.1 - wild-type tau; 2.2 - M123 tau. Isotype control: 3.1 - M123 tau).
Fig. 4c) shows statistics about phosphoepitope frequency (Y axis - percentage of cells displaying phosphoepitopes as % of all transfected cells) among different conditions: 1 - wild-type tau, 2 - M23, 3 - M12, 4 - M123. Left graph - AT8 immunolabeling, right graph - AT 100. Scale: 8 µm.

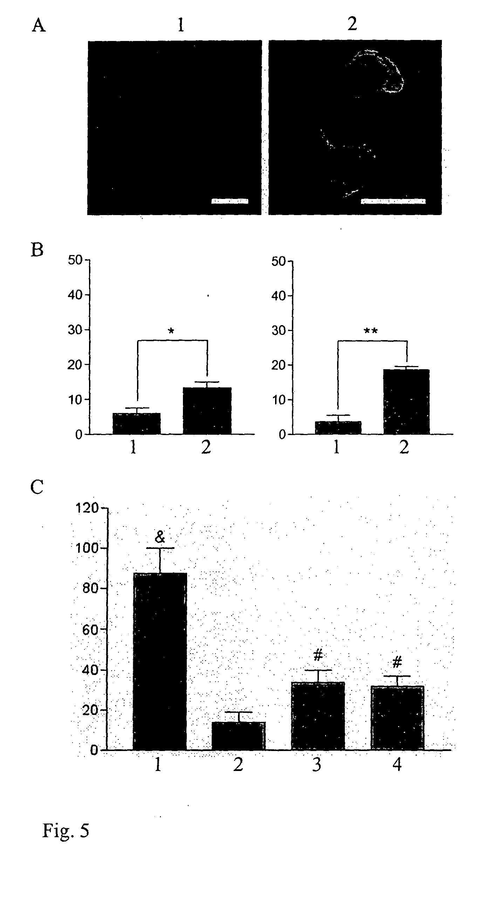

Fig. 5a) shows TUNEL-positive nuclei (red in the coloured original) in control SHSY5Y cells (1), treated with 50 nM staurosporin, and also in M123-transfected neuroblastoma cells (2). A2 shows a cytoplasmic signal from the M123-GFP (green in the original) and a nuclear TUNEL signal (red in the coloured original). Scale: 8 µm.
Fig. 5b). A significant increase in the percentage of TUNEL-positive cells (left graph) is observed in M123-transfected neuroblastoma cells (2) in comparison with wild-type tau transfected cells (1) 48 h. post transfection (* p<0.05). Activated caspase-3 immunoreactivity quantification (right graph) also revealed increased apoptosis in M123-trnasfected cells (2) in comparison with wild-type transfected (1) (** p<0.01). Y axis - percentage of transfected cells.
Fig. 5c). Specific cell-permeable caspase-3 inhibitor, as well as cytochrome c release inhibitor were tested and an increase in cell survival was observed. 1 - wild-type tau, 2 - M123 mutant tau, 3 - M123 mutant plus caspase-3 inhibitor, 4 - M123 mutant plus cytochrome c release inhibitor (& p<0.001; #p<0.05). Y axis - percentage of transfected cells.

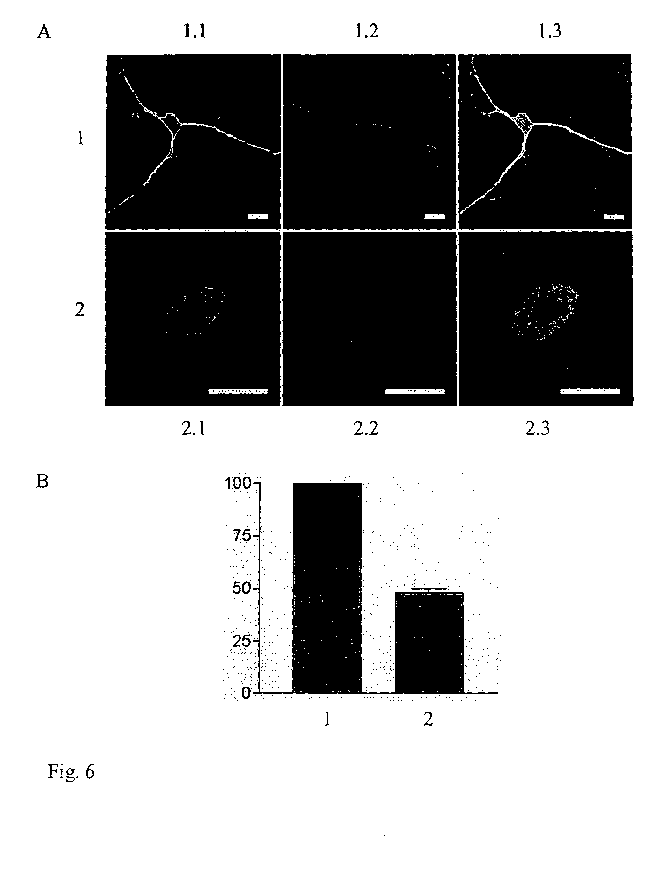

Fig. 6a) shows that the triple mutant M123 decreased the number of process-forming embryonic neurons by 50%, thus demonstrating apparent neurotoxicity. Fig. 6a) shows the presence of tau protein aggregates in the neuronal soma of M123-transfected mutants, as well as the spatial dissociation of tau protein aggregates and cytoskeleton (cytoskeleton labeled with the beta-tubulin III antibody) (1 - wild-type tau, 1.1 - GFP-tau, 1.2 - beta-tubulin III immunolabeling, 1.3 - overlay (yellow in the coloured original, indicating colocalization); 2 - M123 tau, 2.1 - GFP-M123 tau, 2.2 - beta-tubulin III immunolabeling, 2.3 - overlay (the green signal of M123 and the red signal of beta-tubulin are separate from one another, indicating a lack of colocalization in the coloured original).
Fig. 6b). Relative percentage of branching neurons (1 - wild-type tau, 2 - M123 mutant tau; Y axis - relative percentage, numbers normalized to the wild-type neurons).

### DESCRIPTION OF THE TABLES

Table 1 a) shows the polypeptide sequences of the tubulin binding repeats 2 (R2), 3 (R3) and 4 (R4) of human tau 441; Table 1b) shows the corresponding sequences in the mutant TBRs named M1, M2 and M3, respectively. The pluses/minuses in the lowest rows show the polar/non-polar patterns of the TBRs and the mutant TBRs. Minuses indicate non-polar amino acids, pluses are polar amino acid residues.

Table 2 shows that aggregation and AT8-immunoreactivity correlate with toxicity, but not AT100 epitope frequency.

### DESCRIPTION OF THE SEQUENCES

SEQ ID NO.:1 is the tubulin binding repeat 1 of human tau 441.
SEQ ID NO.:2 is the tubulin binding repeat 2 of human tau 441.
SEQ ID NO.:3 is the tubulin binding repeat 3 of human tau 441.
SEQ ID NO.:4 is the tubulin binding repeat 4 of human tau 441.
SEQ ID NO.:5 is the consensus sequence for tubulin binding repeats.
SEQ ID NO.:6 is the tubulin binding repeat 1 of bovine tau.
SEQ ID NO.:7 is the tubulin binding repeat 2 of bovine tau.
SEQ ID NO.:8 is the tubulin binding repeat 3 of bovine tau.
SEQ ID NO.:9 is the tubulin binding repeat 4 of bovine tau.
SEQ ID NO.:10 is the tubulin binding repeat 1 of human MAP2B.
SEQ ID NO.:11 is the tubulin binding repeat 2 of human MAP2B.
SEQ ID NO.:12 is the tubulin binding repeat 3 of human MAP2B.
SEQ ID NO.:13 is the tubulin binding repeat 1 of bovine MAP4.
SEQ ID NO.:14 is the tubulin binding repeat 2 of bovine MAP4.
SEQ ID NO.:15 is the tubulin binding repeat of human MAP4.
SEQ ID NO.:16 is the tubulin binding repeat of rat MAP2.
SEQ ID NO.:17 is the mutated tubulin binding repeat 2 of human tau 441, named M1.
SEQ ID NO.:18 is the mutated tubulin binding repeat 3 of human tau 441, named M2.
SEQ ID NO.:19 is the mutated tubulin binding repeat 4 of human tau 441, named M3.
SEQ ID NO.:20 is the longest N-terminal projection domain of the human splice variants of tau.
SEQ ID NO.:21 is a tau projection domain of the splice variant with exon 3 missing.
SEQ ID NO.:22 is a tau projection domain of the splice variant with exons 2 and 3 missing.
SEQ ID NO.:23 is a tau projection domain of peripheral tau.
SEQ ID NO.:24 is a C-terminal projection domain.
SEQ ID NO.:25 is human tau 441.
SEQ ID NO.:26 is the 372aa Isoform of mouse tau.
SEQ ID NO.:27 is the 430aa Isoform of mouse tau.
SEQ ID NO.:28 is the 374aa Isoform of rat tau.
SEQ ID NO.:29 is the 432aa Isoform of rat tau.
SEQ ID NO.:30 is bovine tau.
SEQ ID NO.:31 is drosophila tau.
SEQ ID NO.:32 is the C. elegans tau-like protein with 453aa.
SEQ ID NO.:33 is the C. elegans tau-like protein with 413aa.
SEQ ID NO.:34 is the tau mutant M2.
SEQ ID NO.:35 is the tau mutant M23.
SEQ ID NO.:36 is the tau mutant M12.
SEQ ID NO.:37 is the tau mutant M123.

### EXAMPLES

The following examples are meant to further illustrate, but not limit the invention. The examples comprise technical features and it will be appreciated that the invention relates also to combinations of the individual technical features presented in this exemplifying section.

The biochemical transition of normal physiological tau, bound to the microtubules, into pathological tau, aggregated in a macromolecular structure, presents a remarkable change in which the protein switches from one type of association to another through a traj ectory involving multiple steps. The transition is even more remarkable in that normal tau has no discernible secondary structure when isolated or produced from recombinant sources. However, the aggregated situation is not unordered, but instead a high β-sheet content exists. It therefore seems likely that the unstructured nature of tau allows multiple interactions with different cellular substructures, and likely also different target proteins that compete in a healthy neuron. The disadvantage of this flexibility is that -under certain conditions- malign interactions might occur. Given the long lifespan of a neuron, these mistargeting events accumulate and culminate in polymeric pathogenic tau fibrils, which process is most probably accelerated by oxidative stress and other environmental factors. In this respect, it is important to note that a high proportion of proteins in the recently sequenced genomes of several species, not in the least *Homo sapiens,* contain large unstructured domains or are even unstructured in full length (Wright and Dyson (1999). *J*. *Mol*. *Biol*., **293,** 321-331). This evolutionary conservation argues for important, yet unknown, cellular roles. In many cases, the lack of structural integrity of these non-globular proteins is alleviated upon binding to a target molecule, which then acts as a folding template. It is apparent that unstructured proteins have a unique functional advantage by this induced-fit mechanism, since it allows precise control over the thermodynamics of the binding process and provides a simple mechanism for inducibility by phosphorylation or through differential interaction with other components of the cellular machinery. The unstructured nature of tau might not only allow modulation of microtubule binding, but the portions of the protein that stay unstructured once tau polymerizes into fibrils also might allow the binding of a large variety of cellular proteins. The large insoluble tau fibrils might provide a highly efficient docking surface for cellular proteins that might then be pathologically recruited from their regulated localization in the healthy neuron, to the unnatural and autonomous fibril surface where they can exert their enzymatic activity outside of normal homeostatic control. This might constitute a pathological "gain-of-function" mechanism, which might be favorable over a "loss-of-function" explanation of tau toxicity as tau's function has been shown to be redundant by the presence of other microtubule-associated-proteins (MAPs), or a mechanical obstruction, *e*.*g*. for organelle transport as mutant tau can be toxic before discernable fibrils are generated. Alternatively or in addition, the tau fibrils could interfere with axonal and cellular transport processes that are essential for maintaining proper cellular functional and synaptic integrity.

### Example 1: Designed tau mutants

We have noted that the amino-acids in the microtubule-binding repeats form an incomplete, alternating pattern of polar and apolar residues (Table 1a). Nature seems to avoid complete alternating patterns of polar and non-polar amino acids, as a screen of all known sequences for proteins shows the least prevalence for sequences of this sort (Broome and Hecht (2000) *J*. *Mol. Biol*. **296,** 961-968). West *et al*. (1999) *Proc. Natl*. *Acad. Sci. USA* **96,** 11211-11216 and Wang *et al*. (2002) *Proc. Natl. Acad. Sci. U S A* **99,** 2760-2765 have shown that isolated peptides consisting of complete alternating patterns of polar and non-polar amino acids spontaneously form β-sheet fibrils similar to amyloid fibrils. Neurofibrillary tangles were not addressed in these publications.

Based on the available pattern in the first three microtubule-binding domains, we have introduced complete alternating pattern of at least 5, and in case of the experiments shown 11 polar and apolar residues by three mutations that were introduced individually and in combinations. This tuning effort produced a stretch of 11 uninterrupted polar/non-polar residues with the described pattern and with minimal perturbation of the native sequence if all three mutations are considered (Table 1b).

We speculated that this manipulation might increase the intrinsic tendency of tau to form a β-sheet structure. We further speculated that this might lead to induced aggregation of tau and might possibly give an effect that is close to the pathological state of tauopathies like non-familiar AD, with all the respective pathological aspects, but possibly in a significantly shorter time period.

Within the microtubule-binding regions TBR2, TBR3 and TBR4 of tau we identified three regions, containing a motif of several successive polar/non-polar aminoacid residues with a slight break in the middle of the motif. In TBR3 additionally there is one proline (P), which is a classic structural breaker. These motifs, although imperfect, allowed - after a limited number of amino acid replacements, deletion and rotations - to achieve a sequence of 11 polar/non-polar aminoacids. In terms of the abbreviations present in this exemplifying section, mutations within region 275-305 are referred as M1, between 306-336 as M2 and between 337-368 as M3. Favourable periodicity in associated tau molecules is achieved by the formation of disulfide bridges through oxidation of cystein at two positions within the tau molecule, thus limiting alternative overlapping of the protein. Preferred polypeptides of the invention are obtainable by modifications of one or more of the motifs 275-305 (numbering according to the longest tau 441, but also all corresponding regions of different splice forms with different numberings, as well as all artificial splice forms with corresponding regions between flexible PGGG motifs are entailed), 306-336, 337-368 and 369 up to the end of the C-terminal, which include one or several of the following: 1. partial or full modification of the amino acid sequence in order to increase the overall number of such polar/non-polar amino acid motifs (e.g. Introduction of 1, 2, 3, 4, 5, 6, 7 or more such motifs) and/or the length of such polar/non-polar amino acid motifs (e.g. extending the length of naturally occurring polar/non-polar amino acid motifs by 1, 2, 3, 4, 5, 6, 7, 8, or even 9 amino acids) 2. elongation or duplication of regions of polar/non-polar amino acids and reduction, preservation, elongation or duplication of the neighbouring regions, as well as removal of one or more PGGGs 3. excision of polar and/or non-polar residues in order to achieve a decrease in the size of a break region, that is a region which interrupts such a polar/non-polar amino acid motif 4. any artificial replacement of whole or partial motifs with different size and/or compositions of artificial polar/non-polar amino acid motifs, not met in tau, but following the pattern of successive polar/non-polar residues, for example 5, 6, 7, 8, 9, 10, 11, 12, 13, 14 or even 15 successive polar/non-polar residues, 5. the inclusion of other beta-sheet inducing sequences, like from silk polypeptide.

In the next sections, the mutants such generated will be abbreviated as follows: M2, M23 (*i.e.* M2+M3), M12 (*i.e.* M1+M2) and M123 (*i*.*e*. M1+M2+M3), while wild-type tau will be referred as "wild-type tau" or WT.

In order to establish the biophysical and neuropathological properties of our rational mutants in comparison with wild-type tau441, several cell types were used as model systems: CHO-cells (chinese hamster ovary fibroblasts), SHSY5Y (human neuroblastoma cell line) and primary hippocampal mouse neurons.

### Example 2: Aggregation and FRAP in CHO cells

Following Effectene-based plasmid DNA transfection with a triple mutant (M123) in CHO cells, formation of morphologically apparent aggregates was observed, while wild-type tau showed normal MT (microtubule) association without aggregates. M2 showed some increased aggregation. 24 hrs post transfection, 3 types of aggregates (Fig. 2a) were visible in double and triple mutant cells, namely: 1. punctate microaggregates (variant 1) 2. bulky aggregates (oval or submembranous ring-like) (variant 2) 3. granular cytosolic accumulation (variant 3) The same picture was observed with the double mutants, but not with wild-type tau (Fig. 2b). Wild-type tau associated with microtubules, while aggregating mutant cells showed no overlap between aggregates and the tubulin cytoskeleton (Fig. 2c). On the contrary - in areas with bulky aggregation a deficit of the normal cytoskeletal configuration was observed. In order to visualize a difference in the biophysical properties of tau and aggregating mutants, FRAP (fluorescent recovery after photobleaching) analysis was performed in CHO-cells 24 hours after transfection, using wild-type tau and the triple (M123) mutant. Tau tagged with GFP represents a perfect tool for aggregation examination in living cells in real time. Apparent differences were found between wild-type tau and the triple mutant. While the immobile fraction of wild-type tau was only about 3.6%, showing extremely high mobility due to diffusion and probably transport with a short half time of about 9 sec., the triple mutant proved to be highly immobile in the cytosol with an immobile fraction of about 88% even 200 sec. after bleaching with a very slow recovery of fluorescence, if any (Fig. 3).

### Example 3: Toxicity and phosphorylation-directed immunocytochemistry in human SHSY5Y neuroblastoma cells

In order to examine the neurotoxicity of the mutant tau variants in human neuronal cell line, SHSY5Y neuroblastomas were used and the cell number of each preparation at 48 and 72 hours post transfection was normalized to the cell number in the same dish at 24 hours. The triple mutant exerted a strong neurotoxicity at 72 h. post transfection, as this effect was already obvious at 48 h. post transfection (Fig. 4a). The same effect was observed by the double mutants. Further, the presence of two specific pathological phosphoepitopes was tested using phosphospecific antibodies - AT8 (S202/T205 specific) and AT100 (T212/S214), the latter being exclusively specific for Alzheimer's disease NFTs and generally considered as the only AD-specific antibody by experts in the field of neuropathology. Although described as being slightly phosphorylated in mitotically active cells like CHO cells and LAN-5 neuroblastoma (Illenberger *et al*. (1998), *Mol*. *Biol*. *Cell*. **9,** 1495-1512), in our SHSY5Y-based ex vivo system the S202/T205 phosphoepitope following AT8 immunolabeling was found to be only very faintly present in very few neuroblastoma cells (9.17±2.3%) 36 h. after transfection with wild-type tau. In the mutants (especially in the double and triple mutants) there was a very strong AT8 labeling, reaching 83.24±5.45% in M123 (Fig. 4c). In contrast, the AT8-detected phosphorylation in CHO cells shows only a slight pattern for wild-type tau, signifying the relative independence of aggregation on phosphorylation (data not shown). Detailed analysis at different timepoints after transfection (6, 12, 18, 24 h.) showed very early initial expression of tau-GFP protein (approx. 9-10 h. post transfection). This was accompanied by early phosphorylation, colocalizing with the initial tau aggregates. Nevertheless, 12 h. post transfection 60% of aggregating cells showed AT8 labeling, compared to 100% at 24 h., supporting the hypothesis of the secondary occurrence of pathologic phosphorylation (Illenberger *et al., supra*). In another experiment, an N-terminal truncated form of the triple mutant (deletion of fragment aa 49-244) was expressed and a similar aggregation pattern was observed. This mutant completely lacked a fragment which contains most phosphorylation sites of the projection domain (including the corresponding sites for AT8, AT100, AT180, AT270), thus confirming the independence of aggregation and phosphorylation. While at 12 h. post transfection AT8 labeled only aggregates, at 24 h. all of the cellular tau (inside and outside aggregates) was found to be phosphopositive. Another phosphoepitope that is highly specific for AD - T212/S214 (recognized by AT 100 antibody), was present especially in double and triple mutants (Fig. 4d). In contrast to AT8, this immunolabeling was not observed in all aggregates, but only in those with bulky rounded or oval shape. This antibody did not label cells with wild-type tau. Interestingly, AT8 labeled double and triple mutants with similar frequency, while AT100 immuostaining increased from M23 to M12 and reached highest levels in M123. Obviously, the formation of the T212/S214 phosphoepitope requires, apart from S202/T205 phosphoepitope formation (Schneider *et al*. (1999) *Biochemistry* **38,** 3549-3558), also a specific conformational modification, apparently achieved with increase of mutant epitope number. In the M123 mutant 22.13±2.87% were also AT180 immunopositive (phosphoepitope T231) vs. none in wild-type tau transfectants (not shown). In order to assess the relative contribution of the aggregation, S202/T205 and T212/S214 phosphorylation to cell toxicity, multiple correlation analyses were performed. The aggregation rate of mutants and S202/T205 phosphorylation revealed high correlation with cell toxicity, while T212/S214 phosphorylation correlated only weakly with cell loss, confirming the primary importance of S202/T205 phosphoepitope formation in cell toxicity and the secondary role of T212/S214 phosphoepitope formation (Table 2).

### Example 4: Apoptotic cell death in SHSY5Y cells

In order to analyse apoptosis induction in SHSY5Y neuroblastoma cells transfected with wild-type and triple mutant tau, the TUNEL method was used to label fragmented nuclear DNA, a characteristic marker of apoptosis. This was combined with the assessment of a second pathomorphological criteria: shrunken cell body with sparse cytosol and rounded cell morphology. Such a combination of criteria is considered sufficient to identify positvely the occurrence of apoptosis (Stadelmann and Lassmann (2000) *Cell Tissue Res.* **301,** 19-31). Since 48 hours after transfection a significant decrease in cell number and apparent cell degeneration is observed, this timepoint was chosen for the comparative analysis. At this timepoint the mode of degeneration, if apoptotic, should have been obvious. Indeed, 13.37±1.6% in the M123 transfected cells showed signs of apoptosis vs. 5.97±1.54% in the normal tau transfectants (*p<0.05, Mann-Whitney U-test) (Fig. 5a,b). Although significantly different, the absolute values do not completely fit with the difference between wild-type tau and M123 cell survival at 48 hours, which is about 25% and more than 40% at 72 hours. In Fig. 5a, apoptotic and non-apoptotic M123-transfected cells are shown, as well as a positive 50 nM staurosporin-treated neuroblastoma control. It should be mentioned, and attention should be taken, that nearly all cells considered apoptotic in M123-transfected neuroblastoma showed pyknotic, but not fragmented nuclear morphology as considered by TUNEL labeling (Fig. 5a). Activated caspase-3 immunolabeling, however, detected 18.66±0.93% apoptotic cells in M123 transfected neuroblastoma cells vs. 3.62±1.87% in wild-type transfected cells (p<0.01, Mann-Whitney U-test). The reason for this atypical apoptosis presentation in our system is unknown and awaits further elucidation. Next, in order to assess the real biological significance of apoptosis in the process of cell elimination, specific cell-permeable caspase-3 inhibitor and cytochrome c release inhibitor (both from Calbiochem, Germany) were used. Cells were treated 24 h. post transfection immediately after counting their reference number and incubated with apoptosis inhibitors until 60 h. post transfection in order to cover the time window with the highest decrease in cell number. Both inhibitors protected cell loss. The caspase-3 inhibitor performed slightly better with a survival effect of 34.03±5.57% vs. 14.12±4.65% in M123 mutant and 87.86±12.4% in normal tau transfected cells (& p<0.001; # p<0.05, One-way analysis of variance (One-way ANOVA) with Bonferroni post test) (Fig. 5c). The cytochrome c release inhibitor protected survival up to 31.72±5.29% (# p<0.05, One-way analysis of variance (One-way ANOVA) with Bonferroni post test). Thus, we showed that apoptosis probably plays a role in mutant tau-induced cell death in a certain population of neuroblastoma cells. Nevertheless, the apoptosis detection results, as well as the effect of specific apoptosis inhibitors, did not explain the amount of cell toxicity completely, indicating alternative toxicity pathways. This information is important in terms of current evidence of apoptotic events in AD brains (Stadelmann *et al*.(1999) *Am. J. Pathol*. **155,** 1459-1466; Lassmann *et al*. (1995) *Acta Neuropathol. (Berl)* **89,** 35-41; Kobayashi *et al*. (2003) *J. Neurol*. *Sci.* **208,** 17-24; and Pompl *et al*. (2003) *Arch. Neurol*. **60,** 369- 376), although the lack of large scale apoptosis in our system also fits to the current understanding about the cell loss in AD and other tauopathies (Jellinger *et al., J, Alzheimers Dis.* **3,** 31-40).

### Example 5: Neurotoxicity of mutant tau in primary mouse neurons

All available data convincingly showed that, among all the mutants we developed, the "triple" mutant M123 reproduced the features of the pathological tau in AD most reliably. In order to determine its neurotoxicity in primary neurons, transfection during seeding was performed with normal tau and M123 triple mutant, using Effectene transfection reagent (Qiagen). 36 h. post transfection, the percentage of normally forming processes neurons was determined and normalized to normal tau. Neurons were identified unequivocally by beta-tubulin III immunoreactivity in order to exclude effects in morphologically similar glial cells present in some preparations. Triple mutant M123 decreased the number of process-forming neurons by 50%, thus demonstrating apparent neurotoxicity (Fig. 6). Another interesting finding was the presence of tau protein aggregates in the neuronal soma of M123-transfected mutants, as well as dissociation of tau protein aggregates and cytoskeleton, labelled with the beta-tubulin III antibody (Fig. 6a).

### Example 6: Pathological effects in transgenic drosophila

Transgenic Drosophila were created using the well known GAL4/UAS-based expression system (Brand and Perrimon (1993) Development **118**: 401-415). A genetic cassette was constructed which allowed inducible overexpression of the triple mutant M123 in a tissue specific manner.
Briefly, Drosophila driver lines, which express the Gal4 protein in a specific tissue/cell type are readily available from the scientific community, for example from the Bloomington Stock Center, Indiana, USA. The expression patterns of the *GAL4* drivers are described in Flybase (http://flybase.bio.indiana.edu).
DNA coding for the M123 triple mutant was cloned downstream of UASs, GAL4-binding sites which assure that overexpression from the UAS-M123 construct is induced only wherever and whenever Gal4 is present. Expression of Gal4, and thus activation of overexpression of the UAS-M123-construct, is a thermodependent process (18 degrees Celsius - very low expression; 25 degrees Celsius - increased expression, 29 degrees Celsius - maximum expression).
To create pUAS-M123, the coding region of M123, tagged with GFP, was cloned as a 2 kb large fragment, restricted with KpnI(blunted)/ApaI from a pcDNA3 vector into the XbaI(blunted)/ApaI sites of pUAS (Brand and Perrimon, 1993). DNA constructs were introduced into *w Drosophila* by germ line transformation. In this way the reporter line UAS-M123 was generated.

To examine the phenotype of flies ectopically expressing the triple mutant M123 under control of different *GAL4* drivers, crosses were set up with a single vial for each transformant. The flies were reared at 18°C.
The crosses between transformants carrying either a wt-tau or a M123-transgene and the ELAV-*GAL4* stock (P{GawB}elav^{C155}) were performed in the following way. Three vials, each containing five pairs of virgin females and males, were set up for each transformant line tested. Crosses were performed at 18°C; progeny were collected.
Overexpression of mutant tau was induced by a temperature shift to 29 degrees Celsius shortly after egg laying. ELAV-GAL4 crossed to UAS-M123 allowed a maximum level of targeted expression in all tissues of the nervous system.
In preliminary experiments we have observed that starting at about 5 days after hatching the overall activity of M123-flies, their speed of moving and adaptation to stress was decreased.
We further expect a decrease in life span and vacuolization in the CNS due to neuronal death upon M123 expression (especially in older animals, over 15 days old) together with specific phosphoepitopes present on the phosphorylation sites of M123, as well as some discrete motor deficit and apparent subcellular aggregates in larvae after strong expression of mutant tau M123.

### Example 7: Pathological effects in transgenic mouse

Since the tau-mutant M123 is neurotoxic in cell-culture within a very short time after expression, we prefer a conditional expression system in transgenic animals, for example in transgenic mice.
The TET-on system of expression in mice (Kistner et al. (1996) Proc Natl Acad Sci USA 93:10933-10938) allows to control the expression of the gene coding for the mutant human tau protein M123. Expression occurs only after the animals are fed with doxycycline (e.g. 2 mg/kg in the water supply).
Briefly, a transactivator mouse line and a reporter mouse line are established by pronuclear injection according to established protocols (Hogan, B., Constantini, F. and Lacy, E. (1995) Manipulating the Mouse Embryo: a laboratory manual (Cold Spring Harbor Lab. Press, Plainview, NY), 2^{nd} Ed.).
The transactivator line is based on a pTet-On vector, which vector produces the reverse Tc-controlled transactivator (rtTA). The vector contains a neuron-specific promoter (e.g. synapsin).
The reporter mouse line is produced using a pTRE-based vector with either wild-type tau (as a control) or the triple tau mutant M123, which has a size of 2 kb when GFP-tagged, cloned in the vector's MCS (multiple cloning site) downstream of a TRE (tet-responsive element)-containing promoter. The two mouse lines are crossed, yielding offspring carrying both transgenes. The presence of both transgenes is confirmed by genotyping via PCR amplification of genomic DNA from tail biopsies and, for example, using primers to detect GFP sequences (when wild-type and mutant tau are GFP-tagged). The number of integrated transgene copies is measured by dot-blot hybridization with an GFP hybridization probe.
Once doxycycline is present, rtTA/docycyline complex binds to the TRE of the reporter construct and triggers transcription, and thereby expression, of GFP-tau (in the control mice) and GFP-M123. To this end, the animals are exposed to 1 to 2 mg/kg doxycycline hydrochloride, dissolved in 5% sucrose, supplied as drinking water and exchanged every 3 days. Expression of wild-type and mutant tau should be induced most preferably when mice are mature, most preferably after 6 month and not earlier that 2 months.
Deficits in learning and memory as tested by standard behavioural tests such as 8-arm radial maze, Morris water maze, shuttle box and others (Reddy DS. Preclinical and clinical behavioral paradigms for testing drugs that affect learning and memory processes. Methods Find Exp Clin Pharmacol. 1998 Apr;20(3):249-77) are expected to be observed, motor deficits (in animals expressing in motor neurons), decreased spontaneous activity and fitness at least 7 days after beginning of expression in the triple mutant-expressing mice in comparison with the control mice with expression of wild-type tau. The lifespan of the M123-expressing mice is also expected to be shortened.
We also expect NFTs formation, specific phosphorylation, cell death and impairment of synaptic function and morphology as judged by electrophysiological and histomorphological methods, as well as astroglial reaction and neuroinflammation ealiest day 2 after expression in the triple mutant expressing animals in comparison with wild-type tau-expressing ones.

### Example 8: Preventing pathological effects in transgenic mice by vaccination

Wild-type tau and the triple tau mutant M123 (poly)peptides are expressed, for example in *E. coli,* and isolated to homogeneity, including a high performance liquid chromatography step. The protein solution is transferred into a suitable buffer, for example 1 x PBS (where 1x PBS stands for 0.15 M NaCl, 0.01 M sodium phosphate, pH 7.5). After 24 h. incubation the suspension is again properly mixed. Wild-type and mutant tau (antigen within the range of 50 µg to 2000 µg per injection depending on the size of the immunization (poly)peptide) are emulsified 1:1 (v/v) with complete Freund's adjuvant for the first immunization, which takes place at day 80 after birth of the double transgenic mouse of Example 7, followed by a boost with the antigen in incomplete Freund's adjuvant at 2 weeks, and monthly thereafter (preferred routes of administration - intradermally and subcutaneously). Wild-type or mutant tau proteins in PBS alone are injected from the fifth immunization onward. Expression of tau and mutant tau is induced at 6 months after birth.

An alternative immunization approach utilizes naked DNA vector injection (Waisman et al. (1996) *Nat. Med. 2*:*899*). Cardiotoxin is injected into the tibialis anterior muscle of the double transgenic mouse of Example 7 starting at day 80 after birth (10 µM per leg). At 1 wk following injection, animals are injected with 100 µg DNA in PBS. DNA is a mammalian expression vector, containing a fragment of 1.4 kb - wild-type or the triple tau mutant M123 under the control of a CMV-promoter. At 4 to 5 days after the first immunization, one animal from each group could be sacrificed, and transcription of the wild-type or mutant tau is verified using RT-PCR on tibialis anterior muscle samples. Thereafter, naked DNA vaccines are given three to five times, with intervals of 6 to 7 days between each injection (Youssef et al. (1998) J Immunol. 161(8):3870-9). Also in this alternative, expression of tau and mutant tau is induced at 6 months after birth.

In the immunized animals the deficits in learning and memory as tested by standard behavioural tests such as 8-arm radial maze, Morris water maze, shuttle box and others (Reddy DS. Preclinical and clinical behavioral paradigms for testing drugs that affect learning and memory processes. Methods Find Exp Clin Pharmacol. 1998 Apr;20(3):249-77) are expected to be significantly milder than the deficits observed without vaccination in Example 7. Also the motor deficits, the decreased spontaneous activity and the decreased fitness observed in the mice of Example 7 at least 7 days after beginning of expression in the triple mutant-expressing mice are expected to be significantly less pronounced and are expected to occur later after the onset of expression in the vaccinated mice than in the unvaccinated mice of Example 7. Also the lifespan of the M123-expressing mice is prolonged in comparison with the unvaccinated mice of Example 7. Also a reduction in the intensity of NFTs formation, specific phosphorylation, cell death and impairment of synaptic function can be observable in the vaccinated mice compared with the unvaccinated mice of Example 7.

### Recapitulation

We have developed a unique rationale for the generation of tau mutants, providing the first ever precise cellular model for studying AD-like tau aggregation and tauopathies as a whole, utilizing a single tau molecule expression model. Our approach works in the absence of artificial coexpression of other enzymes or active substances that induce excessive phosphorylation, as is the case for current model systems. Our method is thus superior as these treatments perturb the cell machinery and homeostasis, rendering the assessment of the influence of tauspecific effects impossible. Thus, using specially optimized mutant tau we were able to recapitulate the most typical and highly specific characteristics of pathological tau in NFT, namely: 1. aggregation 2. cell toxicity and neurotoxicity, gross morphological change 3. pathologic AT8 phosphorylation 4. AD-specific pathological AT100 phosphorylation 5. involvement of apoptosis 6. pathologic AT 180 phosphorylation. Additionally, we confirm the crucial significance of microtubule-binding repeats R1-R4 and especially the first 12 aminoacid residues after each PGGG aminoacid sequence in these repeats in promoting pathological tau aggregation, phosphorylation and toxicity. This novel model presents unprecedented opportunities to determine key molecular events leading to pathologic tau neurotoxicity, as well as the possibility for drug screening in *ex vivo* neuronal and non-neuronal cell models.

### MATERIALS AND METHODS

### Site-directed mutagenesis

Human tau coding sequence (GeneBank accession No. NM_005910) of the 441 aminoacid long-form tau (NCBI protein database, accession number AAC04279) was amplified by PCR, using Deep Vent Polymerase (New England Biolabs) and primers with the following sequences - cgcggatccctatcacaaaccctgcttggccag (BamHI restriction site underlined), agagagcggccgcggcggcatggctgagccccgcca (Not I restriction site underlined). Tau sequence was subcloned in a pcDNA3 vector (Invitrogen GmbH, Germany) with a GFP fused at the N-terminal of tau using a linker consisting of Gly-Gly-Arg-Gly-Gly residues, as part of the NotI restriction site. Point mutations were introduced using 14 cycles of amplification and subsequent Dpnl digestion, using the following primers:

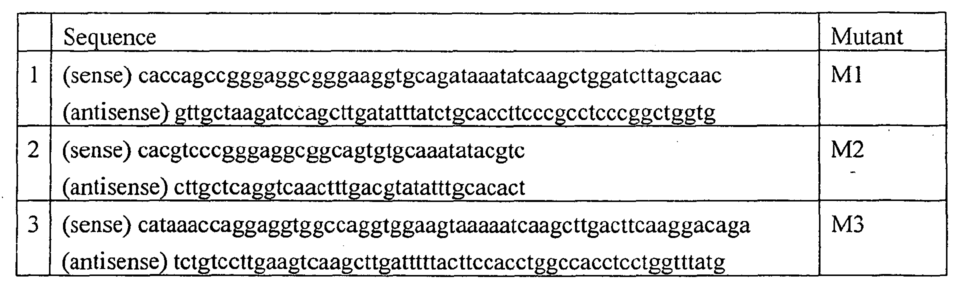

Combinations of different mutations were performed in a sequential way. All constructs were verified by dye-terminated sequencing of the whole coding sequence.

### Cell lines cultures and primary neuronal cultures

CHO (Chinese hamster ovary) fibroblasts, SHSY5Y human neuroblastoma cells were obtained from ATTC, USA. CHO fibroblasts were cultured in BME-based medium (Basal Medium Eagle (GibcoBRL, Invitrogen GmbH, Germany), supplemented 10% fetal calf serum (PAN Biotech GmbH, Germany), 1% penicillin/streptomycin and 1% L-glutamine (all from GibcoBRL, Invitrogen GmbH, Germany). SHSY5Y neuronal cells were grown in RPMI1640 medium (GibcoBRL, Invitrogen GmbH, Germany), supplemented with 10% fetal calf serum (PAN Biotech GmbH, Germany), 1% penicillin/streptomycin. Cells were grown depending on the application in 75 ml. cell culture flasks, 6-well dishes, 12-well dishes, 24-well dishes or 4-well glass-bottom chambers (all from Nunc GmbH&Co. KG, Germany).
Primary neuronal cultures were prepared from C57BL/6 mice. Hippocampi were removed from brains of C57BL/6 embryos (E16) and separated from the meninges. Subsequently they were mechanically dispersed in BME-based neuronal medium (basal medium Eagle, GibcoBRL, Invitrogen GmbH, Germany), 2% B-27 neuronal supplement (GibcoBRL, Invitrogen GmbH, Germany), 1% glucose (Sigma, Germany), 1% fetal calf serum (PAN Biotech GmbH, Germany). Cells were seeded into poly-L-ornithine and laminin (Sigma, Germany) pretreated 4-well chambers (Nunc GmbH&Co. KG, Germany) with either low (50 000 cells/well) or high cell density (200 000 cells/well).

### Transfection procedure

Transfection of cells was performed, using the Effectene Transfection Kit (Qiagen, Germany) according to the manufacturers instructions. Following addition of 4-8 µl enhancer solution, 0.5-1 µg DNA were incubated with 10 µl Effectene and 85 µl transfection buffer. All plasmids were purified using EndoFree Maxi Kit (Qiagen, Germany).
Transfection of neurons was performed during seeding with the Effectene Transfection Kit (Qiagen, Germany) according to the manufacturers instructions. Following addition of 4-8 µl enhancer (as provided by supplier) 0.5-1 µg DNA per 106 cells were incubated with 12 µl Effectene and 85 µl transfection buffer for a period of 30 min. Subsequently, cells were washed in ice-cold PBS, reconstituted in neuronal medium and seeded at a density of 150 000 cells/ chamber in 4-well chamber slides.

### Immunocytochemistry

After fixation in 4% paraformaldehyde (PFA) for 20 minutes at room temperature the preparations were permeabilized with 0.1% Triton X-100 (Sigma, Germany) for 5 min. and blocked with 2% bovine serum albumin (BSA, Sigma, Germany). Cells were incubated with a primary antibody directed against the following proteins at the indicated concentration for 1 h. room temperature in PBS:

| | Antibody | Company | Dilution |
|---|---|---|---|
| 1 | Anti-alpha tubulin (mo, monoclonal) | Sigma, Germany | 1:1000 |
| 2 | Anti-beta-tubulin III (mo, monoclonal) | Sigma, Germany | 1:300 |
| 3 | Anti-C-terminal tau (mo, monoclonal) | Zytomed, Germany | 1:500 |
| 4 | Anti-AT8 tau (mo, monoclonal) | Innogenetics, Belgium | 1:500 |
| 5 | Anti-AT100 tau (mo, monoclonal) | Innogenetics, Belgium | 1:500 |
| 6 | Anti-AT180 tau (mo, monoclonal) | Innogenetics, Belgium | 1:200 |
| 7 | Anti-activated Caspase-3 (rb, polyclonal) | BD Biosci., Germany | 1:500 |
| 8 | Isotype controls | BD Biosci., Germany | 1:100-1:200 |

Secondary goat anti-mouse antibodies, conjugated to either Cy3 or Cy5 (1:300, Jackson ImmunoResearch Laboratories, USA), were incubated for 30 min. at room temperature. The slides were mounted in Mowiol (Hoechst AG, Aventis Pharma Deutschland GmbH, Germany) and stored at 4°C.

Confocal imaging and FRAP (Fluorescent Recovery After Photobleaching) imaging Confocal imaging was performed with a Leica TCS SP2 confocal system fitted with an AOBS (acousto-optical beam splitter) (Leica, Germany). The objectives used were 63x water or oil immersion; adjustments were as follows: Beam Expander 3, PMT - 600-700 V, pinhole 1-1.4 airy disc, zoom - variable. Luminophore excitation was performed with an Argon-ion laser at 488 nm (for GFP), GreNe laser at 543 nm (for Cy3) and HeNe 643 nm (for Cy5).
FRAP was performed on CHO cells 24 h. post transfection in a 4-well chamber with a German borosilicate glass coverslip bottom using a heated 63x oil objective and a heated (36 degrees Celsius) microscopy chamber. Bleaching was performed by repeated continuous (10 frames) scanning at 100% laser power at 488 nm in a 32x zoom, thus defining a bleaching square with 4x4 µm size. Series of images of recovery were acquired 10 sec. after bleaching at 5 sec. intervals with a laser power of 1-4% and a zoom factor of 8x. Intensity measurement was performed using Leica Confocal Software (Leica, Germany), installed on a Windows-based PC.

### Toxicity evaluation

Toxicity of the constructs was analysed in a SHSY5Y-based system. SHSY5Y neuronal cells were transfected with 0.5 µg DNA/dish in 12-well dishes. 24 h. post transfection number of GFP-positive cells were counted in 8 separate fields of view on a Zeiss Axiovert 200 microscope using a 10x air objective. The number of GFP-positive cells in exactly the same fields were repeatedly counted at 48 and 72 hours post transfection and expressed relative to the cell count at 24 hours to compensate for transfection efficiency differences between dishes.

### Statistics

Data were analyzed by One-way ANOVA, p-value adjustment for repeated testing by the Bonferroni method and Mann-Whitney U-test, performed using GraphPad Prism version 3.00 for Mac OSX operating system (GraphPad Software, San Diego California USA, www.graphpad.com) and Microsoft Excel Software (Microsoft Corporation, USA).

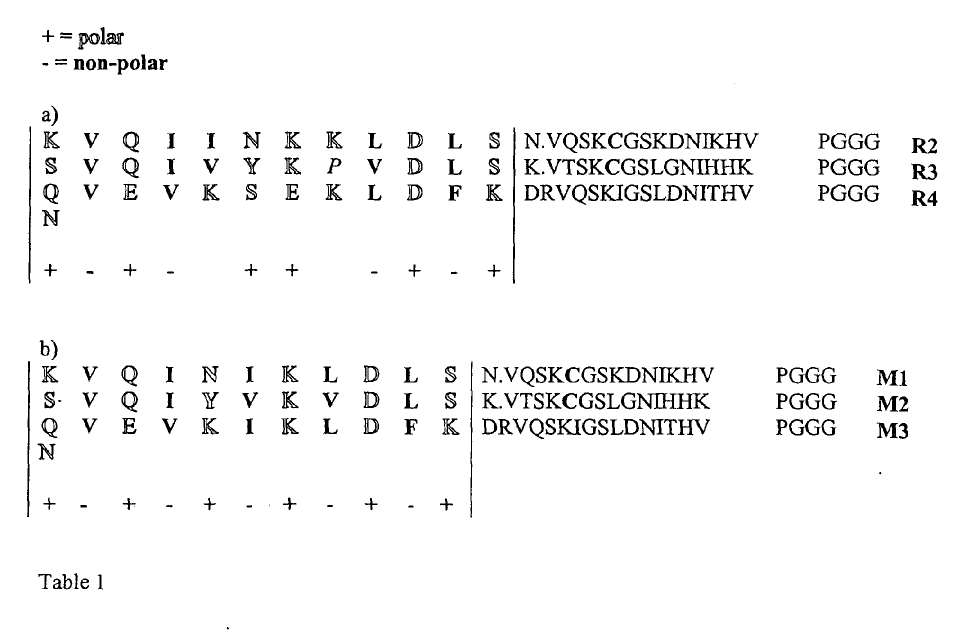

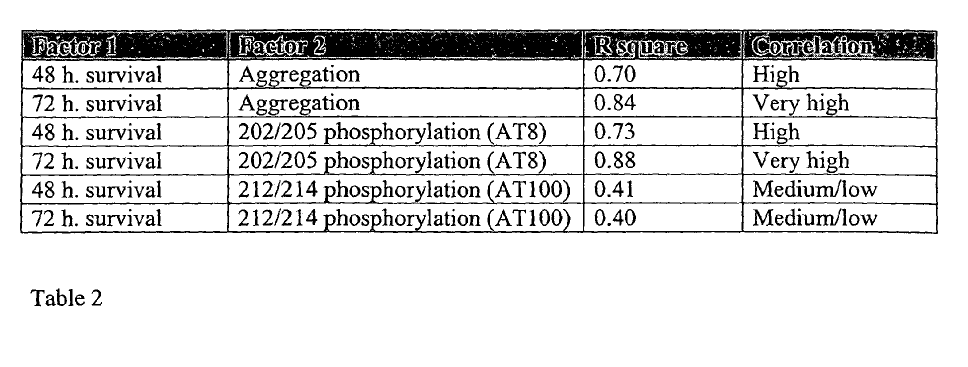

## Claims

1. A polypeptide comprising
a) at least one modified microtubule binding domain of a tau protein wherein the modified microtubule binding domain comprises a region of at least 7 alternating polar and non-polar amino acids; or
b) at least two microtubule binding domains of a human tau protein, wherein
b1) one microtubule binding domain is modified and comprises two regions of at least 6 alternating polar and non-polar amino acids, or
b2) two microtubule binding domains are modified and both comprise a region of at least 6 alternating polar and non-polar amino acids; or
c) at least three microtubule binding domains of a human tau protein, wherein
c1) two microtubule binding domains are modified and one of the modified microtubule binding domains contains two regions of at least 5 alternating polar and non-polar amino acids, and the second of the modified microtubule binding domains comprises one region of at least 5 alternating polar and non-polar amino acids, or
c2) three microtubule binding domains are modified and all three comprise a region of at least 5 alternating polar and non-polar amino acids;
which polypeptide is capable of displaying increased aggregation *ex vivo* and/or causing increased neurotoxicity and/or cell toxicity compared with the wild type human tau protein.

2. The polypeptide of alternative a) of claim 1, wherein said region of alternating polar and non-polar amino acids consists of 8, 9, 10, 11, 12, 13, 14 or 15 alternating polar and non-polar amino acids, preferably of 9, 10, 11, 12 or 13 alternating polar and non-polar amino acids and more preferably of 10, 11 or 12 alternating polar and non-polar amino acids.

3. The polypeptide of any one of claims 1 to 2, wherein the polar residues of said region of alternating polar and non-polar amino acids are selected from the group consisting of Ser, Arg, Lys, Asp, Glu, Asn, Gln, His, Thr, Tyr, Trp, Gly, Ala and Cys, preferably selected from the group consisting of Ser, Arg, Lys, Asp, Glu, Asn, Gln, and Thr, and more preferably selected from the group consisting of Ser, Lys, Asp and Gln.

4. The polypeptide of any one of claims 1 to 3, wherein the non-polar residues of said region of alternating polar and non-polar amino acids are selected from the group consisting of Ile, Val, Leu, Phe, Gly, Ala and Cys, preferably selected from the group consisting of Ile, Val, Leu and Phe, and more preferably selected from the group consisting of Ile, Val and Leu.

5. The polypeptide of any one of claims 1 to 4, wherein at least two of the polar residues of said region of alternating polar and non-polar amino acids are charged amino acid residues.

6. The polypeptide of claim 5, wherein at least one amino acid residue is a histidine residue, a lysine residue or an arginine residue and at least one amino acid residue is an aspartic acid residue or a glutamic acid residue.

7. The polypeptide of any one of claims 1 to 6, wherein the region of alternating polar and non-polar amino acids starts 17 to 35 amino acids upstream of a flexible linker region, preferably 23 to 31 amino acids upstream of said flexible linker region, preferably 24 to 30 amino acids upstream of said flexible linker region, more preferably 25 to 29 amino acids upstream of said flexible linker region, and most preferably 26 to 28 amino acids upstream of said flexible linker region.

8. The polypeptide of any one of claims 1 to 7, wherein a cysteine residue is located between 7 and 13 amino acid residues upstream of a flexible linker region, preferably 8 to 12 amino acids upstream, more preferably 9 to 11 amino acids upstream and most preferably 10 amino acids upstream of said flexible linker region.

9. The polypeptide of any one of claims 1 to 8, wherein the modified microtubule binding domain differs in 2 to 5 amino acid residues from any one of SEQ ID NOs 1-16, preferably in 3 to 4 amino acid residues.

10. The polypeptide of claim 1 which comprises, and preferably contains, two, preferably three and more preferably four microtubule binding domains.

11. The polypeptide of claim 1 which comprises, and preferably contains, two, preferably three and more preferably four modified microtubule binding domains.

12. The polypeptide of claim 11, wherein at least two regions of alternating polar and non-polar amino acids start at the same number of amino acid residues +/- 2 amino acid residues upstream of a flexible linker region, preferably at the same number of amino acid residues +/- 1 amino acid residues upstream of said flexible linker region, and more preferably at the same number of amino acid residues upstream of said flexible linker region.

13. The polypeptide of any one of claims 1 to 12, wherein the polypeptide further comprises a polypeptide sequence having at least 90%, preferably 95%, more preferably 98% and most preferably 100% identity with any one of the polypeptide sequences SEQ ID No.s 20-24, preferably located N-terminally from the modified microtubule binding domain.

14. The polypeptide of any one of claims 1 to 13 which is a full-length tau polypeptide, wherein the polypeptide sequence corresponding to amino acids 275-286, 306-317 or 337-348 of human tau 441 (SEQ ID NO:25) comprises at least 7 alternating polar and non-polar amino acids, and preferably contains, 7, 8, 9, 10 and more preferably 11 alternating polar and non-polar amino acids,
or wherein the polypeptide sequences corresponding to amino acids 275-286 and 306-317; 275-286 and 337-348; or 306-317 and 337-348 each comprise at least 6 alternating polar and non-polar amino acids (double mutants), preferably wherein the polypeptide sequences corresponding to amino acids 275-286 and 306-317; 275-286 and 337-348; or 306-317 and 337-348 each contain 7, 8, 9, 10 and more preferably 11 alternating polar and non-polar amino acids;
or wherein the polypeptide sequences corresponding to amino acids 275-286 and 306-317 and 337-348 (triple mutants) each comprise at least 5 alternating polar and non-polar amino acids, preferably wherein the polypeptide sequences corresponding to amino acids 275-286 and 306-317 and 337-348 each contain 6, 7, 8, 9, 10 and more preferably 11 alternating polar and non-polar amino acids.

15. The polypeptide of any one of claims 1 to 14, wherein the region of alternating polar and non-polar amino acids starts with a polar amino acid.

16. The polypeptide of any one of claims 1 to 15 which comprises a polypeptide with the sequence SED ID NO. 17-19, particularly which is the polypeptide with the sequence SEQ ID NO. 34-37.

17. The polypeptide of any one of claims 1 to 16 further comprising a peptide sequence for purification and/or detection purposes.

18. A nucleic acid molecule comprising a nucleic acid sequence encoding a polypeptide according to any one of claims 1 to 17.

19. An expression cassette comprising the nucleic acid molecule of claim 18, preferably further comprising expression control sequences operatively linked to a nucleic acid encoding a polypeptide according to any one of claims 1 to 17.

20. A host cell, particularly a mammalian, non-human cell, inside or outside of the animal body or a human cell outside of the human body, comprising a polypeptide according to any one of claims 1 to 17, a nucleic acid according to claim 18 and/or an expression cassette according to claim 19.

21. A transgenic animal comprising a host cell according to claim 20.

22. A diagnostic kit for the detection of the aggregated form of human tau protein comprising
a) an antibody which specifically binds to the aggregated form of human tau protein, and
b) a polypeptide according to any one of claims 1 to 17 or an extract of the host cell according to claim 20 or of the transgenic animal of claim 21 as a positive control for the aggregated form of human tau protein.

23. A polypeptide according to any one of claims 1 to 17 or an expression cassette according to claim 19 or an extract of the host cell according to claim 20 or an extract of the transgenic animal of claim 21 for use in medicine and/or veterinary medicine, in particular for use in a method according to claim 31.

24. A screening method for identifying molecules which alters a phenotype induced by a modified microtubule binding domain of a human tau protein, said method comprising the following steps:
a) providing a host cell according to claim 20 or a transgenic animal according to claim 21; and
b) adding a molecule to be tested; and
c) identifying the molecules the addition of which has led to a change of the induced phenotype in cell culture or in the transgenic animal, which change is indicative of molecules which alter the induced phenotype.

25. The screening method of claim 24, wherein the induced phenotype is neurotoxicity, tau-aggregation, tau-phosphorylation or cell toxicity.

26. The screening method of claim 25, wherein the change of the induced phenotype in cell culture or in the transgenic animal is an amelioration of the induced phenotype.

27. A method for identifying proteins interacting with the aggregated and/or pathogenic form of tau, said method comprising the steps of:
a) providing a host cell according to claim 20 or a transgenic animal according to claim 21; and
b) identifying the polypeptides/proteins which interact with the polypeptide according to any one of claims 1 to 17.

28. The method of claim 27, wherein the interacting polypeptides/proteins are identified by biochemical purification of the polypeptide according to any one of claims 1 to 17, in particular of a polypeptide according to claim 17, and subsequent identification of binding polypeptides/proteins by immunological, biochemical and/or biophysical methods, in particular by mass-spectroscopical methods.

29. The method of claim 27, wherein the interacting polypeptides/proteins/RNAs are identified by protein-RNA and/or protein-protein-interaction assays, in particular by a two-hybrid assay or a microscopy-based protein-protein interaction assay, preferably FRET and FRAP.

30. The method of claims 28 or 29 further comprising the identification of polypeptides/proteins interacting with wildtype human tau protein as a negative control step.

31. A method for the generation of conformation-specific anti-tau-antibodies which selectively recognize human tau in the aggregated conformation, said method comprising the administration of a polypeptide according to any one of claims 1 to 17 or of an extract of the host cell according to claim 20 or an extract of the transgenic animal of claim 21 to an animal to induce an immune response of a subject.

32. The method of claim 31 for the generation of monoclonal and/or recombinant antibodies.

33. The method of claim 32 further comprising a negative selection step for antibodies which also recognize wildtype tau protein.

34. Use of a polypeptide according to any one of claims 1 to 17 or an expression cassette according to claim 19 or an extract of the host cell according to claim 20 or an extract of the transgenic animal of claim 21 for the preparation of a composition for immunizing a subject.

35. Use of a polypeptide according to any of claims 1 to 17 in in-vitro screening methods.

36. Use of a polypeptide according to any one of claims 1 to 17 or of the nucleic acid according to claim 18 or of the expression cassette according to claim 29 for the induction of neurofibrillary tangles in cells, preferably in cells in cell culture.

37. Use of a polypeptide according to any one of claims 1 to 17 for the isolation of proteins which interact with neurofibrillary tangles.

38. Use of a polypeptide according to any one of claims 1 to 17 or of an extract of the host cell according to claim 20 or an extract of the transgenic animal of claim 21 for the generation of antibodies which selectively recognize human tau in the aggregated conformation.

39. Use of a polypeptide comprising a polypeptide according to claims 1-17 and a second polypeptide, for which second polypeptide one desires to find an interaction partner, in a protein-protein-interaction screen and/or assay.

40. Use of the nucleic acid of claim 18 or the expression cassette of claim 19 for the generation of a transgenic animal.
